# EUROPÄISCHE PATENTSCHRIFT

(11) **EP 3 443 103 B1**
(45) Veröffentlichungstag und Bekanntmachung des Hinweises auf die Patenterteilung: **12.11.2025**
(21) Anmeldenummer: 17720379.1
(22) Anmeldetag: 12.04.2017
(51) Int. Cl.: C12N 15/82, C12N 9/02

(54) **KERNKODIERTE MÄNNLICHE STERILITÄT DURCH MUTATION IN CYTOCHROM P450 OXIDASE**
NUCLEUS-ENCODED MALE STERILITY THROUGH MUTATION IN CYTOCHROME P450 OXIDASE
STÉRILITÉ MÂLE CODÉE DANS LE NOYAU PAR MUTATION DANS LA CYTOCHROME P450 OXYDASE

(30) Priorität: 12.04.2016 DE 102016106656
(43) Veröffentlichungstag der Anmeldung: 20.02.2019
(73) Patentinhaber: KWS SAAT SE & Co. KGaA, 37574 Einbeck (DE)
(72) Erfinder: BORCHARDT, Dietrich, 37574 Einbeck (DE); CZARNECKI, Olaf, 13086 Berlin (DE); MECHELKE, Wolfgang, 37574 Einbeck (DE)
(74) Vertreter: Michalski Hüttermann & Partner Patentanwälte mbB
(86) Internationale Anmeldenummer: PCT/EP2017/058815
(87) Internationale Veröffentlichungsnummer: WO 2017/178541

(56) Entgegenhaltungen:
- VESNA DJUKANOVIC ET AL: "Male-sterile maize plants produced by targeted mutagenesis of the cytochrome P450-like gene ( MS26 ) using a re-designed I- Cre I homing endonuclease", THE PLANT JOURNAL, vol. 76, no. 5, 5 December 2013 (2013-12-05), GB, pages 888 - 899, XP055218454, ISSN: 0960-7412, DOI: 10.1111/tpj.12335
- A. MARK CIGAN ET AL: "Targeted mutagenesis of a conserved anther-expressed P450 gene confers male sterility in monocots", PLANT BIOTECHNOLOGY JOURNAL, vol. 15, no. 3, 1 March 2017 (2017-03-01), GB, pages 379 - 389, XP055401700, ISSN: 1467-7644, DOI: 10.1111/pbi.12633
- H. LI ET AL: "Cytochrome P450 Family Member CYP704B2 Catalyzes the -Hydroxylation of Fatty Acids and Is Required for Anther Cutin Biosynthesis and Pollen Exine Formation in Rice", THE PLANT CELL, vol. 22, no. 1, 1 January 2010 (2010-01-01), US, pages 173 - 190, XP055401749, ISSN: 1040-4651, DOI: 10.1105/tpc.109.070326
- DATABASE UniProt [online] 14 October 2015 (2015-10-14), "SubName: Full=Uncharacterized protein {ECO:0000313|EMBL:KMT20292.1};", XP002773258, retrieved from EBI accession no. UNIPROT:A0A0J8D3D0 Database accession no. A0A0J8D3D0
- M. MORANT ET AL: "CYP703 Is an Ancient Cytochrome P450 in Land Plants Catalyzing in-Chain Hydroxylation of Lauric Acid to Provide Building Blocks for Sporopollenin Synthesis in Pollen", THE PLANT CELL, vol. 19, no. 5, 1 May 2007 (2007-05-01), US, pages 1473 - 1487, XP055401645, ISSN: 1040-4651, DOI: 10.1105/tpc.106.045948
- KIM SUNG SOO ET AL: "Sporopollenin monomer biosynthesis in arabidopsis", JOURNAL OF PLANT BIOLOGY, BOTANICAL SOCIETY OF KOREA, SEOUL, KR, vol. 56, no. 1, 8 February 2013 (2013-02-08), pages 1 - 6, XP035344579, ISSN: 1226-9239, [retrieved on 20130208], DOI: 10.1007/S12374-012-0385-3

## Beschreibung

### GEBIET DER ERFINDUNG

Die vorliegende Anmeldung betrifft das Gebiet der Vereinfachung von arbeitsaufwendigen Züchtungsprogrammen mittels molekularbiologischer Methoden, Markertechnologie und der Gentechnik. Insbesondere werden Pflanzen offenbart, die durch spontane Mutation einer Genregion im Kerngenom einen im homozygoten Zustand, kernkodierten männlich sterilen Phänotyp aufweisen, der sich dadurch auszeichnet, dass die Mutation im Gegensatz zu CMS (*cytoplasmic male sterility*) durch eine rezessive Merkmalsausprägung erhalten wird und somit eine parallele Erhaltung von sterilen und fertilen Genotypen in Züchtungsprogrammen entfällt. Diesbezüglich offenbart die vorliegende Anmeldung Pflanzen, insbesondere Zuckerrübe oder Kartoffel, in der mittels Markertechnologie eine Mutation in einem Cytochrom P450 Oxidase (*CYPgst*) Gen nachgewiesen wurde, die zu der oben genannten Merkmalsausprägung führt sowie das entsprechende Verfahren zur Identifizierung dieser Mutation. Zusätzlich werden ein CYPgst Protein, ein DNA-Molekül enthaltend das mutierte Gen, das zu oben genannter Merkmalsausprägung führt, ein rekombinante DNA-Molekül, das das Wildtypgen, einen Promotor zur spezifischen Expression dieses Gens oder eines heterologen Gens in Blüten und/oder Früchten von Pflanzen und/oder eine Nukleotidsequenz kodierend für Inhibitoren des CYPgst Gens sowie entsprechende Vektoren und Wirtszellen offenbart.

Des Weiteren betrifft die vorliegende Anmeldung gentechnisch veränderte Pflanzen, die einen rezessiven, kernkodierten männlich sterilen Phänotyp durch Inhibierung der Expression des CYPgst Gens zeigen, die entsprechenden Inhibitoren sowie Verfahren zur Inhibierung des Gens und Verfahren zur Restauration der Fertilität. Die Anmeldung betrifft ferner die Verwendung der Pflanzen in Hybridzüchtungsverfahren, Resistenzzüchtungsverfahren und/oder in der Saatgutproduktion. Ferner sind Samen oder Nachkommen, Organe, Pflanzenteile, Gewebe oder Zellen der dieser Pflanze sowie deren Verwendung miterfasst.

### HINTERGRUND DER ERFINDUNG

Zur gesteuerten Erzeugung genetischer Variation werden in Zuckerrüben (*Beta vulgaris* subsp. *vulgaris*)*,* aber auch in allen anderen Kulturpflanzenarten, Kreuzungen durchgeführt. Dabei werden Pollen tragende Antheren manuell aus noch geschlossenen Blüten des Samenelters entfernt. Die Bestäubung erfolgt dann ebenso manuell durch Aufbringen von Pollen des Pollendonors auf die Narbe des Samenelters. Alternativ kann nach Entfernung der Antheren an Blüten des Samenelters eine Bestäubung auch dadurch erreicht werden, dass der Pollenspender in räumlicher Nähe zum Samenelter zum Blühen und zur Pollenschüttung gebracht wird. In jedem Fall handelt es sich um außerordentlich arbeitsintensive Protokolle, die durchaus fehleranfällig sind, denn z.B. eine ungewollt nur partielle Entfernung der Antheren einer Blüte kann zur Selbstbestäubung führen.

Die Erzeugung von (kommerziellem) Hybridsaatgut erfolgt gegenwärtig häufig durch Einkreuzung der Sameneltern-Komponente in CMS (*cytoplasmic male sterility*)*-*tragende Genotypen und anschließende Rückkreuzung, um den genetischen Anteil der Samenelter-Komponente zu erhöhen. Die resultierende männlich sterile Samenelter-Komponente kann im Anschluss großflächig angebaut werden und die Bestäubung erfolgt durch in räumlicher Nähe wachsende Pollenspender (*topcross* Verfahren). Da CMS dominant maternal vererbt wird, erfordert die Verwendung von CMS-Linien die parallele Bereithaltung von fertilen *Maintainer-*Linien (also nicht in CMS eingelagerte O-Typen), die die Bestäubung der CMS-Linien sicherstellen. Dies bringt einen hohen Planungs- und Produktionsaufwand sowie eine komplexe Logistik mit sich. Beispielsweise werden kommerzielle Zuckerrüben gegenwärtig als Dreifach-Hybride erzeugt, um Saatgut von ausreichend hoher Qualität zu produzieren. Die Produktion von Hybriden in den Zuchtprogrammen ist ebenfalls kosten- und arbeitsintensiv und wird gegenwärtig durch Aufstellen von Trennwänden verwirklicht.

In zahlreichen Pflanzenarten gibt es Beschreibungen von Linien bzw. Genotypen, die eine natürlich vorkommende kernkodierte männliche Sterilität *ms-*(*male sterile*) zeigen. Verursacht wird diese in der Regel durch spontane Mutation eines Gens im Kerngenom und erhalten wird diese Mutation durch eine rezessive Merkmalsausprägung. Die Verwendung von Genotypen mit kernkodierten männlich sterilen Phänotypen ist geeignet, Teile von Züchtungsprozessen zu vereinfachen und/oder diese für die Erzeugung von Hybridsaatgut einzusetzen. Dabei haben die männlich sterilen Phänotypen den Vorteil, dass sie für Kreuzungen nicht manuell von Antheren befreit werden müssen und eine parallele Erhaltung von fertilen und sterilen Genotypen entfällt, denn durch Selbstbefruchtung spalten sich in jedem Vermehrungsschritt die heterozygoten Genotypen wiederum in fertile und sterile Individuen auf.

Aufgabe der vorliegenden Erfindung war daher Mittel und Verfahren zur Verwendung der kernkodierten männlichen Sterilität in Nutzpflanzen, insbesondere Zuckerrübe zur Verfügung zu stellen. Die Aufgabe wird erfindungsgemäß durch die in den Ansprüchen und in der Beschreibung gekennzeichneten Ausführungsformen gelöst.

### ZUSAMMENFASSUNG DER ERFINDUNG

Die vorliegende Anmeldung betrifft das Gebiet der Vereinfachung von arbeitsaufwendigen Züchtungsprogrammen, der Markertechnologie sowie der Gentechnik. Sie betrifft Pflanzen, die durch Mutation in einem DNA-Segment umfassend eine Cytochrom P450 Oxidase (CYPgst) einen kernkodierten, rezessiven, männlich sterilen Phänotyp aufweist. Das CYPgst Gen sowie die Mutation wurden mittels Markertechnologie und molekularbiologischen Methoden identifiziert. Da die Mutation durch die rezessive Merkmalsausprägung erhalten bleibt und durch Selbstbefruchtung in jeden Vermehrungsschritt heterozygote Genotypen wiederum in fertile und sterile Genotypen aufgespalten werden, entfällt die parallele Erhaltung von fertilen *Maintainer-*Linien*.* Zusätzlich können die Erkenntnisse dazu genutzt werden, transgene Pflanzen mit einem kernkodierten, rezessiven, männlich sterilen Phänotyp zu generieren und um die Fertilität zu restaurieren.

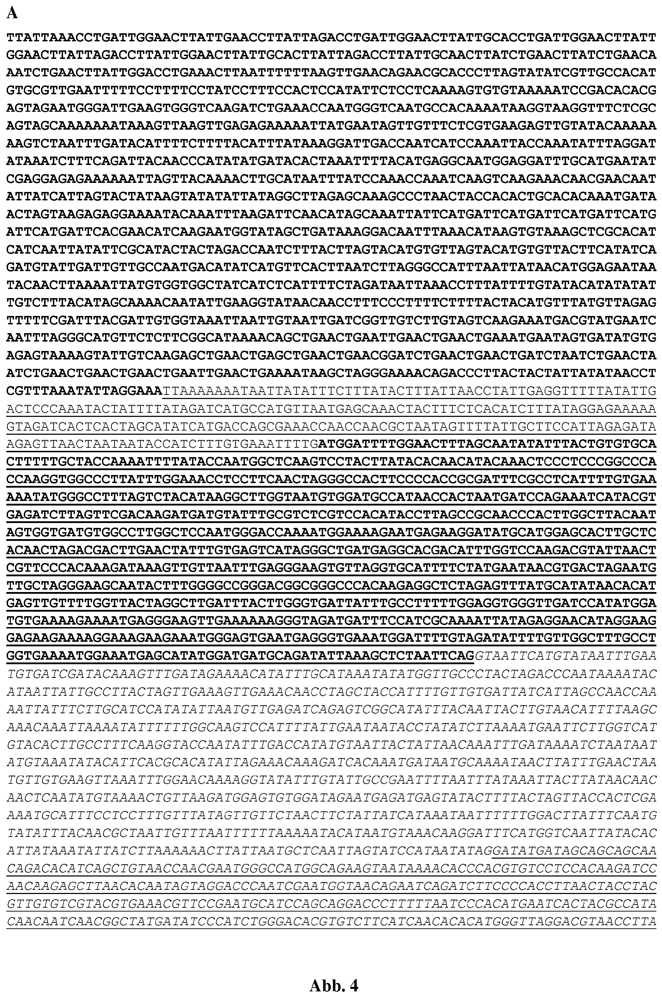

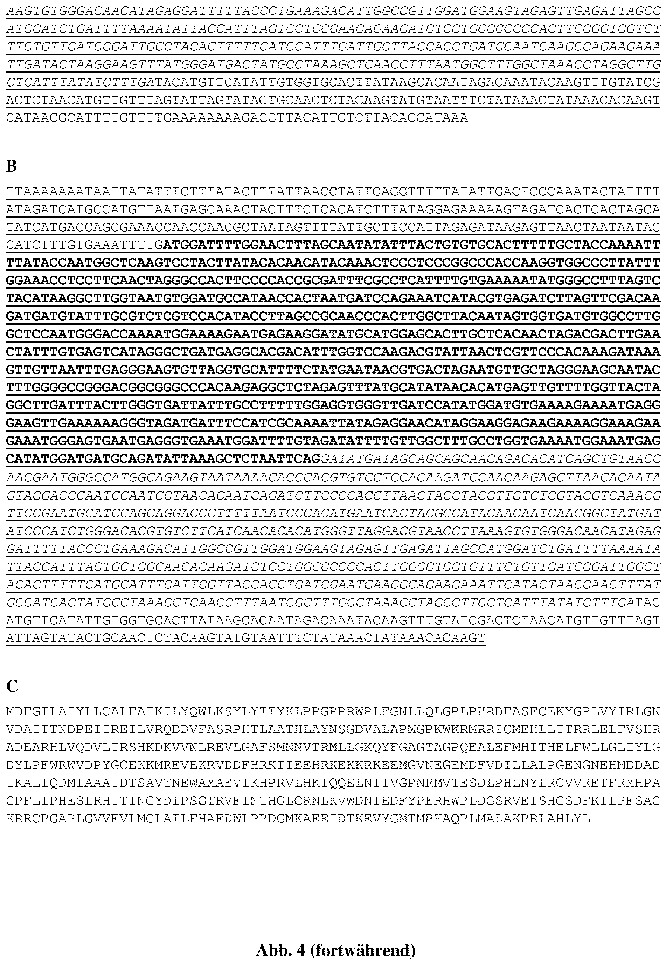

Die vorliegende Anmeldung offenbart im Folgenden
eine Pflanze, insbesondere Nutzpflanze, die einen rezessiven, kernkodierten männlich sterilen Phänotyp zeigt, dadurch gekennzeichnet, dass der Phänotyp mit einer Mutation, welche von dem endogenen Cytochrom P450 Oxidase (CYPgst) Gen umfasst wird, oder mit der Abwesenheit bzw. im Vergleich zu einer entsprechenden (männlich fertilen) Wildtyppflanze geringen Gehalt bzw. Aktivität eines funktionsfähigen CYPgst Proteins, das durch das Wildtypgen von CYP*gst* kodiert wird, korreliert, dadurch gekennzeichnet, dass es sich bei dem nicht-mutierten CYP*gst* Gen a) um das Gen *Bv*CYP*gst* aus *Beta vulgaris* handelt, das vorzugsweise eine der in SEQ ID Nr.: 1 oder 2 gezeigte Nukleotidsequenz umfasst bzw. die in SEQ ID Nr.: 3 gezeigte Aminosäuresequenz kodiert, oder um dessen Homolog, Analog oder Ortholog, b) um das Gen *St*CYP*gst* aus *Solanum tuberosum* handelt, das vorzugsweise eine der in SEQ ID Nr.: 12 oder 13 gezeigte Nukleotidsequenz umfasst bzw. die in SEQ ID Nr.: 14 gezeigte Aminosäuresequenz kodiert, oder um dessen Homolog, Analog oder Ortholog, oder c) um das Gen *Zm*CYP*gst* aus *Zea mays* handelt, das vorzugsweise eine der in SEQ ID Nr.: 9 oder 10 gezeigte Nukleotidsequenz umfasst bzw. die in SEQ ID Nr.: 11 gezeigte Aminosäuresequenz kodiert, oder um dessen Homolog, Analog oder Ortholog;
außerdem eine solche Pflanze, die heterozygot für die Mutation und männlich fertil oder homozygot für die Mutation und männlich steril ist, wobei in der sterilen Pflanze die Ausbildung von funktionellem Pollen unterbunden, bevorzugt vollständig unterbunden ist;
außerdem eine solche Pflanze, wobei das CYP*gst* Gen mindestens in geschlossenen Blüten und Früchten exprimiert wird;
außerdem eine solche Pflanze, wobei die Mutation die Transkription und/oder Translation eines funktionsfähigen Proteins verhindert, vorzugsweise wobei es sich bei der Mutation um eine Deletion, Addition, Insertion oder Substitution in der kodierenden Nukleotidsequenz des CYP*gst* Gens, einem Splicing-Signal oder in einer regulatorischen Sequenz, vorzugsweise der Promotorsequenz, des CYP*gst* Gen handelt. In einer bevorzugten Ausführungsform weist das Nukleinsäuremolekül eine Mutation auf, die auch in der Nukleotidsequenz gemäß SEQ ID Nr.: 8 im Vergleich mit dem Wildtypgen der SEQ ID Nr.: 1 zu finden ist. Insbesondere kann es sich bei der Mutation um eine Deletion zwischen den Nukleotidpositionen 1560 und 2095 der SEQ ID Nr.: 1 oder entsprechenden Positionen der SEQ ID Nr.: 12 oder 9 handeln. Die Deletion kann eine Länge von mindestens 20, 30 oder 50 konsekutiven Basenpaaren, bevorzugt von mindestens 100, 150, 200 oder 250 konsekutiven Basenpaaren und besonders bevorzugt von mindestens 300, 400 oder 500 konsekutiven Basenpaaren aufweisen. In einer besonders bevorzugten Ausführungsform umfasst das Nukleinsäuremolekül eine Nukleotidsequenz gemäß SEQ ID Nr.: 8. In einer weiteren bevorzugten Ausführungsform weist das Nukleinsäuremolekül eine Punktmutation in der Nukleotidsequenz der SEQ ID Nr. 1 gemäß der Tabelle 1 auf, vorzugsweise zwischen den Nukleotidpositionen 1560 und 2095 der SEQ ID Nr.: 1;
außerdem eine solche Pflanze, wobei in *Beta vulgaris,* vorzugsweise *Beta vulgaris* subsp. *vulgaris,* die Deletion durch Abwesenheit einer oder beider der Markerloci sle5983d14 (Amplifikationsprodukt der Primer mit SEQ ID Nr.: 4 und 5) und sle5983d17 (Amplifikationsprodukt der Primer mit SEQ ID Nr.: 6 und 7) und durch Anwesenheit eines ubiquitären Markers nachgewiesen werden kann;
außerdem eine solche Pflanze, wobei in *Beta vulgaris,* vorzugsweise *Beta vulgaris* subsp. *vulgaris,* das Gen in einem Segment auf Chromosom 1 zwischen den Markerloci sxn2151s01 und s1e3305s02 lokalisiert ist, wobei die sxn2151s01-Markersequenz dargestellt in SEQ ID Nr. 24 und die sle3305s02-Markersequenz dargestellt in SEQ ID Nr. 26 das Vorhandensein des *gst*-Locus anzeigen und die sxn2151s01-Markersequenz dargestellt in SEQ ID Nr. 25 und die sle3305s02-Markersequenz dargestellt in SEQ ID Nr. 27 die Referenzsequenz anzeigen;
außerdem eine solche Pflanze, wobei das Segment etwa 50 bis 5000 kbp groß ist, vorzugsweise 100 bis 1000 kbp, mehr bevorzugt 100 bis 500 kbp und besonders bevorzugt 200 bis 250 kbp;
außerdem eine solche Pflanze, wobei es sich bei dem nicht-mutierten Gen um das funktionelle Gen *BvCYPgst* aus *Beta vulgaris,* vorzugsweise *Beta vulgaris* subsp. *vulgaris* handelt oder ein funktionelles homologes, analoges oder orthologes Gen einer anderen Kultur- und Nutzpflanze;
außerdem eine solche Pflanze, wobei es sich bei dem homologen, analogen oder orthologen Gen um ein Gen aus aus *Zea mays,* das vorzugsweise eine der in SEQ ID Nr.: 9 oder 10 gezeigte Nukleotidsequenz umfasst bzw. die in SEQ ID Nr.: 11 gezeigte Aminosäuresequenz kodiert, aus *Solanum tuberosum,* das vorzugsweise eine der in SEQ ID Nr.: 12 oder 13 gezeigte Nukleotidsequenz umfasst bzw. die in SEQ ID Nr.: 14 gezeigte Aminosäuresequenz kodiert, aus *Triticum aestivum,* das vorzugsweise die in SEQ ID Nr.: 15 gezeigte Aminosäuresequenz kodiert, aus *Helianthus annuus,* das vorzugsweise die in SEQ ID Nr.: 16 gezeigte Aminosäuresequenz kodiert, aus *Hordeum vulgare,* das vorzugsweise die in SEQ ID Nr.: 17 gezeigte Aminosäuresequenz kodiert, aus *Brassica napus,* das vorzugsweise die in SEQ ID Nr.: 18 gezeigte Aminosäuresequenz kodiert, aus *Brassica oleracea,* das vorzugsweise die in SEQ ID Nr.: 19 gezeigte Aminosäuresequenz kodiert, aus *Brassica rapa,* das vorzugsweise die in SEQ ID Nr.: 20 gezeigte Aminosäuresequenz kodiert, aus *Glycine max,* das vorzugsweise die in SEQ ID Nr.: 21 gezeigte Aminosäuresequenz kodiert, aus *Gossypium,* das vorzugsweise die in SEQ ID Nr.: 22 gezeigte Aminosäuresequenz kodiert, aus *Sorghum bicolor,* das vorzugsweise die in SEQ ID Nr.: 23 gezeigte Aminosäuresequenz kodiert, handelt;
außerdem eine solche Pflanze, wobei das nicht-mutierten Gen (Wildtypgen) eine Nukleotidsequenz aufweist, ausgewählt aus der Gruppe bestehend aus einer:
   (a) Nukleotidsequenz, die die in SEQ ID Nr.: 1 oder SEQ ID Nr.: 2 gezeigte Nukleotidsequenz oder ein funktionelles Fragment davon aufweist (siehe bspw. Abb. 4A und 4B);
   (b) Nukleotidsequenz, die die in SEQ ID Nr.: 3 gezeigte Aminosäuresequenz kodiert;
   (c) Nukleotidsequenz, die in der Lage ist, an eine Nukleotidsequenz komplementär zu einer Nukleotidsequenz gemäß (a) oder (b) unter stringenten Bedingungen zu hybridisieren;
   (d) Nukleotidsequenz, die eine Aminosäuresequenz kodiert, die gegenüber der Aminosäuresequenz gemäß SEQ ID Nr.: 3 Abweichungen in Form von Aminosäure-Deletionen, Substitutionen, Additionen und/oder Insertionen in der Aminosäuresequenz und vorzugsweise eine Sequenzidentität von mindestens 60% über die gesamte Aminosäuresequenz aufweist;
   (e) Nukleotidsequenz, die ein Protein mit der gleichen enzymatischen Aktivität kodiert, wie das Protein, das durch eine Nukleotidsequenz nach einem aus (a) bis (d) kodiert wird; und
   (f) Nukleotidsequenz, die mindestens 200 oder 400, bevorzugt mindestens 600 oder 800, besonders bevorzugt mindestens 1000 konsekutive Nukleotide aus dem Promotor der Nukleinsäuresequenz von SEQ ID Nr.: 1 von Nukleotidpositionen 1 bis 1518, bevorzugt von Nukleotidpositionen 518 bis 1518, besonders bevorzugt von Nukleotidpositionen 1318-1518 oder eine Sequenz, die an diesen Bereich hybridisiert, umfasst, wobei die Nukleotidsequenz in der Lage ist, die Expression des Gens bzw. eines heterologen Nukleinsäuremoleküls, das operativ mit der Nukleotidsequenz verknüpft ist, spezifisch in geschlossenen Blüten und/oder Früchten zu steuern;
   eine Nukeotidsequenz gemäß (c), (d) oder (e) ist beispielsweise eine Nukleotidsequenz, die die in SEQ ID Nr.: 12 oder SEQ ID Nr.: 13 gezeigte Nukleotidsequenz oder ein funktionelles Fragment davon aufweist, oder die die in SEQ ID Nr.: 14 gezeigte Aminosäuresequenz kodiert. Weiterhin ist eine Nukeotidsequenz gemäß (c), (d) oder (e) beispielsweise eine Nukleotidsequenz, die die in SEQ ID Nr.: 9 oder SEQ ID Nr.: 10 gezeigte Nukleotidsequenz oder ein funktionelles Fragment davon aufweist, oder die die in SEQ ID Nr.: 11 gezeigte Aminosäuresequenz kodiert. Ferner ist eine Nukeotidsequenz gemäß (c), (d) oder (e) beispielsweise eine Nukleotidsequenz, die eine Aminosäuresequenz ausgewählt aus der Gruppe bestehend aus SEQ ID Nr.: 15-23 kodiert;
außerdem ein Nukleinsäuremolekül oder rekombinantes DNA-Molekül, das eine der oben genannten Nukleotidsequenz umfasst;
außerdem ein solches rekombinantes DNA-Molekül, das (i) einen Promotor mit einer Nukleotidsequenz wie in [10] (f) definiert umfasst, der mit einem heterologen Nukleinsäuremolekül operativ verknüpft ist, oder (ii) eine kodierende Nukleotidsequenz wie in [10] (a) - (e) definiert umfasst, die operativ mit einem heterologen Promoter verknüpft ist, der vorzugsweise in der Lage ist, die Expression der Nukleotidsequenz spezifisch in geschlossenen Blüten und/oder Früchten zu steuern;
außerdem ein solches rekombinantes DNA-Molekül umfassend eine Nukleotidsequenz, die eine shRNA (*small hairpin RNA*)*,* siRNA *(small interfering RNA*)*,* Antisense-RNA, Sense-RNA oder doppelsträngige RNA kodiert, die nach Expression in einer Pflanzenzelle oder nach Einbringen in eine Pflanzenzelle zur Inhibierung der Expression des funktionellen (nicht-mutierten) CYPgst Gens führt. In einer bevorzugten Ausführungsform weist die Nukleotidsequenz mindestens 15, 16, 17, 18, 19 oder 20, bevorzugt mindestens 21, 22, 23, 24 oder 25, besonders bevorzugt mindestens 30, 35, 40, 45 oder 50, und ganz besonders bevorzugt mindestens 100, 200, 300, 500 oder 1000 konsekutive Nukleotide der SEQ ID Nr.: 1, 2, 9, 10, 12 oder 13 in Sense- oder Antisense-Orientierung oder von mindestens einem Exon der SEQ ID Nr.: 1, 9 oder 12 in Sense- oder Antisense-Orientierung auf. Dabei erstreckt sich das Exon 1 der SEQ ID Nr.: 1 von der Nukleotidposition 1762-2679 und das Exon 2 der SEQ ID Nr.: 1 von der Nukleotidposition 3507-4142. Das Exon 1 der SEQ ID Nr.: 12 erstreckt sich von der Nukleotidposition 1762-2032, das Exon 2 der SEQ ID Nr.: 12 von der Nukleotidposition 2449-3161 und das Exon 3 der SEQ ID Nr.: 12 von der Nukleotidposition 4032-4694. Das Exon 1 der SEQ ID Nr.: 9 erstreckt sich von der Nukleotidposition 2001-2927 und das Exon 2 der SEQ ID Nr.: 9 von der Nukleotidposition 3018-3683. In einer weiteren bevorzugten Ausführungsform weist die Nukleotidsequenz mindestens 15, 16, 17, 18, 19 oder 20, bevorzugt mindestens 21, 22, 23, 24 oder 25, besonders bevorzugt mindestens 30, 35, 40, 45 oder 50, und ganz besonders bevorzugt mindestens 100, 200, 300, 500 oder 1000 konsekutive Nukleotide auf, welche in der Lage sind, spezifisch an eine wie in [10] oder [16] definierte Nukleotidsequenz zu hybridisiert;
außerdem ein solches Nukleinsäuremolekül, das eine Nukleotidsequenz mit einer Mutation in Form einer Deletion, Addition, Insertion oder Substitution umfasst, wobei diese Mutation dazu führt, dass kein funktionsfähiges CYP*gst* Protein synthetisiert wird. Vorzugsweise ist die Mutation in der kodierenden Nukleotidsequenz des CYP*gst* Gens, einem Splicing-Signal oder in einer regulatorischen Sequenz des CYP*gst* Gens, vorzugsweise in dem Promotor des CYP*gst* Gen, lokalisiert. In einer bevorzugten Ausführungsform weist das Nukleinsäuremolekül eine Mutation auf, die auch in der Nukleotidsequenz gemäß SEQ ID Nr.: 8 zu finden ist. Insbesondere kann es sich bei der Mutation um eine Deletion zwischen den Nukleotidpositionen 1560 und 2095 der SEQ ID Nr.: 1 oder entsprechenden Positionen der SEQ ID Nr.: 12 oder 9 handeln. Die Deletion kann eine Länge von mindestens 20, 30 oder 50 konsekutiven Basenpaaren, bevorzugt von mindestens 100, 150, 200 oder 250 konsekutiven Basenpaaren und besonders bevorzugt von mindestens 300, 400 oder 500 konsekutiven Basenpaaren aufweisen. In einer besonders bevorzugten Ausführungsform umfasst das Nukleinsäuremolekül eine Nukleotidsequenz gemäß SEQ ID Nr.: 8. In einer weiteren bevorzugten Ausführungsform weist das Nukleinsäuremolekül eine Punktmutation in der Nukleotidsequenz der SEQ ID Nr. 1 gemäß der Tabelle 1 auf, vorzugsweise zwischen den Nukleotidpositionen 1560 und 2095 der SEQ ID Nr.: 1;
außerdem ein solches Nukleinsäuremolekül von mindestens 15, 16, 17, 18, 19 oder 20, bevorzugt mindestens 21, 22, 23, 24 oder 25, besonders bevorzugt mindestens 30, 35, 40, 45 oder 50, und ganz besonders bevorzugt mindestens 100, 200, 300, 500 oder 1000 konsekutive Nukleotide der SEQ ID Nr.: 1, 2, 9, 10, 12 oder 13 in Sense- and/oder Antisense-Orientierung oder von mindestens einem Exon der SEQ ID Nr.: 1, 9 oder 12 in Sense- oder Antisense-Orientierung. Dabei erstreckt sich das Exon 1 der SEQ ID Nr.: 1 von der Nukleotidposition 1762-2679 und das Exon 2 der SEQ ID Nr.: 1 von der Nukleotidposition 3507-4142. Das Exon 1 der SEQ ID Nr.: 12 erstreckt sich von der Nukleotidposition 1762-2032, das Exon 2 der SEQ ID Nr.: 12 von der Nukleotidposition 2449-3161 und das Exon 3 der SEQ ID Nr.: 12 von der Nukleotidposition 4032-4694. Das Exon 1 der SEQ ID Nr.: 9 erstreckt sich von der Nukleotidposition 2001-2927 und das Exon 2 der SEQ ID Nr.: 9 von der Nukleotidposition 3018-3683. In einer besonderen Ausführungsform erstreckt sich das Nukleinsäuremolekül über mindestens ein Intron der SEQ ID Nr.: 1, der SEQ ID Nr.: 9 oder der SEQ ID Nr.: 12, d.h. das Nukleinsäuremolekül umfasst aufeinanderfolgend i) mindestens ein Nukleotid vom 3'-Ende des Exons 1 der SEQ ID Nr.: 1 (vorzugsweise letztes Nukleotid des Exons 1 der SEQ ID Nr.: 1 in 5'-3'-Richtung; entsprechend Nukleotid an Position 583 der SEQ ID Nr.: 2) und mindestens ein Nukleotid vom 5'-Ende des Exons 2 der SEQ ID Nr.: 1 (vorzugsweise erstes Nukleotid des Exons 2 der SEQ ID Nr.: 1 in 5'-3'-Richtung; entsprechend Nukleotid an Position 584 der SEQ ID Nr.: 2), ii) mindestens ein Nukleotid vom 3'-Ende des Exons 1 der SEQ ID Nr.: 12 (vorzugsweise letztes Nukleotid des Exons 1 der SEQ ID Nr.: 12 in 5'-3'-Richtung; entsprechend Nukleotid an Position 271 der SEQ ID Nr.: 13) und mindestens ein Nukleotid vom 5'-Ende des Exons 2 der SEQ ID Nr.: 12 (vorzugsweise erstes Nukleotid des Exons 2 der SEQ ID Nr.: 12 in 5'-3'-Richtung; entsprechend Nukleotid an Position 272 der SEQ ID Nr.: 13), iii) mindestens ein Nukleotid vom 3'-Ende des Exons 2 der SEQ ID Nr.: 12 (vorzugsweise letztes Nukleotid des Exons 2 der SEQ ID Nr.: 12 in 5'-3'-Richtung; entsprechend Nukleotid an Position 984 der SEQ ID Nr.: 13) und mindestens ein Nukleotid vom 5'-Ende des Exons 3 der SEQ ID Nr.: 12 (vorzugsweise erstes Nukleotid des Exons 3 der SEQ ID Nr.: 12 in 5'-3'-Richtung; entsprechend Nukleotid an Position 985 der SEQ ID Nr.: 13), oder iv) mindestens ein Nukleotid vom 3'-Ende des Exons 1 der SEQ ID Nr.: 9 (vorzugsweise letztes Nukleotid des Exons 1 der SEQ ID Nr.: 9 in 5'-3'-Richtung; entsprechend Nukleotid an Position 927 der SEQ ID Nr.: 10) und mindestens ein Nukleotid vom 5'-Ende des Exons 2 der SEQ ID Nr.: 9 (vorzugsweise erstes Nukleotid des Exons 2 der SEQ ID Nr.: 9 in 5'-3'-Richtung; entsprechend Nukleotid an Position 928 der SEQ ID Nr.: 10). In einer weiteren bevorzugten Ausführungsform weist die Nukleotidsequenz mindestens 15, 16, 17, 18, 19 oder 20, bevorzugt mindestens 21, 22, 23, 24 oder 25, besonders bevorzugt mindestens 30, 35, 40, 45 oder 50, und ganz besonders bevorzugt mindestens 100, 200, 300, 500 oder 1000 konsekutive Nukleotide auf, welche in der Lage ist, spezifisch an eine wie in [10] oder [16] definierte Nukleotidsequenz zu hybridisiert;
außerdem ein solches Oligonukleotid, vorzugsweise mit einer Länge von maximal 50 Nukleotiden, das ein solches Nukleinsäuremolekül oder ein Nukleinsäuremolekül, welches in der Lage ist, spezifisch an eine Nukleotidsequenz gemäß SEQ ID Nr.: 8 zu hybridisiert, umfasst und/oder vorzugsweise eine der folgenden Nukleotidsequenzen aufweist:
   (i) SEQ ID Nr.: 4, 6 oder einem Komplement davon, oder
   (ii) SEQ ID Nr.: 5, 7 oder einem Komplement davon;
einen Vektor, vorzugsweise Pflanzenvektor umfassend ein solches DNA-Molekül bzw. Nukleinsäuremolekül; ;
außerdem einen solchen Vektor, wobei das DNA-Molekül bzw. Nukleinsäuremolekül als Transgen in der Lage ist ein funktionelles CYP*gst* zu exprimieren und vorzugsweise genetisch gekoppelt ist mit einem weiteren Transgen, welches die Weitergabe des DNA-Moleküls bzw. Nukleinsäuremolekül über den Pollen verhindert, vorzugsweise wobei der Vektor bzw. das Transgen des Weiteren eine Expressionskassette aufweist, die zur einer Markierung der Samen führt, vorzugsweise durch Fluoreszenz-Markierung;
außerdem eine Wirtszelle, vorzugsweise Pflanzenzelle enthaltend ein solches rekombinantes DNA-Molekül bzw. Nukleinsäuremolekül;
außerdem ein CYP*gst* Protein, das von einer wie oben definierten Nukleotidsequenz kodiert wird oder ein funktionelles und/oder immunologisch aktives Fragment davon; vorzugsweise handelt es sich bei dem CYP*gst* Protein um a) die Aminosäuresequenz ausgewählt aus der Gruppe bestehend aus SEQ ID Nr.: 3, SEQ ID Nr.: 11 und SEQ ID Nrs.: 14-23 oder b) eine Aminosäuresequenz, welche mit der Aminosäuresequenz gemäß SEQ ID Nr.: 3 zu mindestens 80%, 82%, 84%, 86% oder 88%, bevorzugt zu mindestens 90%, 91%, 92%, 93%, 94% oder 95%, besonders bevorzugt zu mindestens 96%, 97%, 98%, 99% oder 99,5%, vorzugsweise über die Volllänge, identisch ist;
außerdem einen Antikörper, der spezifisch an das CYP*gst* Protein oder Fragment davon bindet;
außerdem einen Kit umfassend ein solches DNA-Molekül bzw. Nukleinsäuremolekül, Oligonukleotid, Vektor, CYP*gst* Protein oder Fragment davon und/oder Antikörper, und gegebenenfalls Reagenzien für nukleinsäurebasierende oder immunologische Nachweisverfahren;
außerdem ein Verfahren zur Herstellung einer Pflanze, insbesondere Nutzpflanze, die einen rezessiven, kernkodierten, im homozygoten Zustand männlich sterilen Phänotyp zeigt, dadurch gekennzeichnet, dass die Expression des CYPgst Gens inhibiert wird;
außerdem ein solches Verfahren, dadurch gekennzeichnet, dass das Verfahren einen Schritt des Einbringens des besagten rekombinanten DNA-Moleküls, des Nukleinsäuremoleküls oder des Vektors beispielsweise mittels Agrobacterium-Transformation, T-DNA-Tagging, homologe Rekombination, Mutagenese wie TILLING sowie gezielte Mutagenese z.B. durch Einsatz von Zinkfinger-Nukleasen, von TALE- (*Transcription Activator-like Effector*) Nukleasen und des CRISPR/Cas Systems umfasst, infolgedessen die Expression des Gens beispielsweise durch RNAi oder Co-Suppression oder aufgrund der eingebrachten Mutation inhibiert wird;
außerdem ein solches Verfahren zur Restauration der Fertilität einer solchen Pflanze oder einer Pflanze erhältlich durch das oben genannte Verfahren umfassend das Einbringen eines funktionellen CYP*gst* Gens in die Pflanze;
außerdem ein solches Verfahren, wobei das CYP*gst* Gen mittels einer solchen rekombinanten DNA oder eines Vektorseingebracht wird oder durch Kreuzung einer Pflanze, die das CYP*gst* Wildtypgen oder ein funktionellen CYP*gst* Gen, vorzugsweise im homozygoten Zustand trägt; optional kann nach der Kreuzung eine Selektion auf Anwesenheit des CYP*gst* Wildtypgens oder des funktionellen CYP*gst* Gens in der Nachkommengeneration erfolgen;
außerdem eine Pflanze enthaltend Pflanzenzellen und/oder erhältlich durch ein oben genanntes Verfahren;
außerdem ein Organ, Pflanzenteil, Gewebe oder eine Zelle dieser Pflanze;
außerdem ein Samen oder Nachkommen einer solchen Pflanze, wobei der Samen oder die Nachkommen dieoben definierte Mutation aufweist und/oder ein rekombinantes DNA-Molekül bzw. Nukleinsäuremolekül oder einen Vektor;
außerdem ein Verfahren zur Identifizierung einer solchen Pflanze durch Nachweis einer Mutation in dem CYPgst Gen bzw. eines Markers, der mit der Mutation gekoppelt ist;
außerdem eine Verwendung eines solchen DNA-Moleküls bzw. Nukleinsäuremolekül, Nukleinsäuremoleküls, Oligonukleotids, Vektors, CYP*gst* Proteins oder Fragments davon, Antikörpers, und/oder Kits zur Identifizierung einer solchen Pflanze, in der Herstellung einer rezessiven, kernkodierten, männlich sterilen Pflanze, in der Herstellung einer Pflanze mit restaurierter Fertilität, in der Herstellung einer Hybridpflanze, in Resistenzzuchtprogrammen, oder zur Saatgutproduktion;
außerdem eine solche Verwendung des DNA-Moleküls bzw. des oben definierten Promotors zur spezifischen Expression von heterologen Nukleinsäuremolekülen in Blüten und/oder Früchten von Pflanzen;
außerdem eine solche Verwendung einer solchen Pflanze, Organ, Pflanzenteil, Gewebe oder Zelle, Samen oder Nachkommen oder einer durch ein solches Verfahren identifizierten oder durch Verwendung erhältlichen Pflanze oder deren Gewebe, Zelle, Nachkommen oder Samen in der Herstellung von Nahrungsmitteln, Werkstoffen, Arzneimitteln bzw. Vorstufen davon, Diagnostika, Kosmetika, Feinchemikalien, Zucker, Sirup, Bioethanol oder Biogas;
außerdem ein Nahrungsmittel, Futtermittel oder Werkstoff enthaltend eine solche Pflanze, Organ, Pflanzenteil, Gewebe oder Zelle, Samen oder Nachkommen oder eine durch ein solches Verfahren identifizierten oder durch Verwendung erhältlichen Pflanze oder deren Gewebe, Zelle, Nachkommen oder Samen;
außerdem eine solche Verwendung einer solchen Pflanze zur Züchtung oder der Herstellung einer Nachkommenpflanze, wobei der kernkodierte männlich sterile Phänotyp zur rekurrenten Selektion genutzt wird.

Zunächst werden einige der in dieser Anmeldung verwendeten Begriffe nachfolgend näher erläutert:
Der Ausdruck "Chromosomensegment", sowie Variationen der Begriffe wie "chromosomales Segment" oder "Segment auf Chromosom" werden wenn nicht anders angegeben äquivalent verwendet und bezeichnen einen spezifischen chromosomalen DNA-Abschnitt eines bestimmten Chromosoms, der mindestens ein Gen umfasst.

Das "CYP*gst* Gen" bzw. das "Wildtyp Gen von CYP*gst*" kodiert für das "CYPgst Protein", welches eine Rolle in der Ausbildung vitaler Pollen spielt, denn eine Mutation in CYPgst führt zu männlich sterilen Pflanzen durch Unterbindung der Ausbildung von funktionellem Pollen. Experimentell wurde dies in der Zuckerrübe (*Beta vulgaris* subsp. *vulgaris*) nachgewiesen. Durch Homologie Vergleiche konnte ein Ähnlichkeit zu dem CYP703 Gen aus *Arabidopsis thaliana* festgestellt werden, welches nach aktuellem Stand der Technik (Morant et al., The Plant Cell, 19 (2007), 1473-1487) in der Synthese des Sporopollenins (Hauptbestandteil vitaler Pollen) eine essentielle Funktion erfüllt und vorzugsweise die Umwandlung von mittelkettigen gesättigten Fettsäuren in die entsprechenden einfach-hydroxylierten Fettsäuren katalysiert, mit einer bevorzugten Hydroxylierung von Laurinsäure an der C-7 Position. Die Ausschaltung des CYP703 Gens in *Arabidopsis thaliana* führte zu einer partiellen männlichen Sterilität. Die Menge an Pollen war zwar reduziert aber es konnte funktionsfähiger Pollen gebildet werden. Daher scheint das Gen aus *Beta vulgaris* subsp. *vulgaris* eine andere Funktion zu übernehmen, denn eine Ausschaltung führt hier zu einer männlichen Sterilität der Pflanze. Ohne an eine Theorie gebunden zu sein erscheint es plausibel, dass das CYP*gst* Gen einer anderen Klasse der CYP Gene zuzuordnen ist bzw. in Kulturpflanzen, insbesondere Nutzpflanzen wie Zuckerrübe eine andere Funktion oder Bedeutung hat als in der niedrigen Modellpflanze *Arabidopsis thaliana*; siehe auch die Diskussion in Beispiel 1. Der Fachmann kann weitere CYP*gst* Proteine Datenbanken unter Verwendung geeigneter Suchprofile und Computerprogramme für das Screening nach homologen Sequenzen bzw. für Sequenzvergleiche entnehmen. Eine mögliche Überprüfung, ob die identifizierten Gene die gleiche Funktion wie das CYP*gst* Gen in *Beta vulgaris* subsp. *vulgaris* erfüllen, kann durch eine Wiederherstellung der Funktion von CYPgst in einer männlich sterilen Zuckerrübenpflanze durch heterologe Expression der identifizierten Gene überprüft werden, d.h. durch Restauration der Fertilität durch das Transgen.

Ohne an eine bestimmte Theorie gebunden zu sein, ist es allerdings auch nicht auszuschließen, dass das CYP*gst* Gen aus *Beta vulgaris* subsp. *vulgaris* und das CYP703 Gen aus *Arabidopsis thaliana* der gleichen CYP-Familie angehören und somit die gleiche oder zumindest eine ähnliche Funktion in der Synthese des Sporopollenins erfüllen, dass jedoch in Kulturpflanzen, die durch jahrelange gezielte Selektion und Kreuzungen hinsichtlich u.a. Ertrag, Schädlingsresistenz, Toleranz gegen abiotische Stressfaktoren, sowie Gehalts pflanzlicher Inhaltsstoffe optimiert wurden, die Fähigkeit zur Kompensation des fehlenden Sporopollenins abhandengekommen ist, und dass somit die Abwesenheit von Sporopollenin dazu führt, dass die Ausbildung von Pollen unterbunden wird und die Pflanze somit männlich steril ist.

Der Begriff "*gst*-Locus" bezeichnet erfindungsgemäß eine genomische DNA-Region in einer Pflanze, insbesondere Nutzpflanze, in der eine Mutation mit einer rezessiv vererbten, kernkodierten männliche Sterilität korreliert, wobei die Mutation das Cytochrom P450 Oxidase (CYP*gst*) Gen umfasst und dazu führt, dass in einer betroffenen, die Mutation im *gst*-Locus enthaltenen und insbesondere für die Mutation homozygoten Pflanze der Gehalt bzw. die Aktivität eines funktionellen CYPgst Proteins im Vergleich zu einer entsprechenden (männlich fertilen), den Wildtyp-Locus enthaltenen Pflanze (Wildtyppflanze) geringer ist oder vollständig abwesend. Typischerweise wird durch die Mutation im CYP*gst* Gen, die Transkription und/oder Translation eines funktionsfähigen CYP*gst* Proteins verhindert.

Unter dem Begriffe "eng-flankierend" wird verstanden, dass zwei Loci (beispielsweise zwei Marker (Markerloci)) auf einer Genkarte weniger als 15 cM, weniger als 12 cM, weniger als 10 cM, weniger als 8 cM, weniger als 7 cM, weniger als 6 cM, weniger als 5 cM, weniger als 4 cM, weniger als 3 cM, weniger als 2 cM, weniger als 1 cM, weniger als 0,5 cM, weniger als 0,2 cM, weniger als 0,1 cM, weniger als 0,05 cM entfernt voneinander sind.

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d. h. Basenpaarungen mit diesem eingeht. Standardverfahren zur Hybridisierung sind beispielsweise in Sambrook et al., Molecular Cloning: A Laboratory Manual 3rd Ed. Cold Spring Harbor Laboratory Press, Cold Spring Harbor, NY, 2001 beschrieben. Vorzugsweise wird darunter verstanden, dass mindestens 60%, weiter bevorzugt mindestens 65%, 70%, 75%, 80% oder 85%, besonders bevorzugt 90%, 91%, 92%, 93%, 94%, 95%, 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. Ionenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen und Homologen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85%, mindestens 90% oder mindestens 95% Sequenzidentität aufweisen. Stringente Hybridisierungsbedingungen sind beispielsweise: Hybridisieren in 4 x SSC bei 65°C und anschließendes mehrfaches Waschen in 0,1 x SSC bei 65°C für insgesamt etwa 1 Stunde. Der hier verwendete Begriff "stringente Hybridisierungsbedingungen" kann auch bedeuten: Hybridisieren bei 68°C in 0,25 M Natriumphosphat, pH 7,2, 7% SDS, 1 mM EDTA und 1% BSA für 16 Stunden und anschließendes zweimaliges Waschen mit 2 x SSC und 0,1% SDS bei 68°C. Bevorzugt findet eine Hybridisierung unter stringenten Bedingungen statt.

"Komplementäre" Nukleotidsequenz bedeutet bezogen auf eine Nukleinsäure in Form einer doppelsträngigen DNA, dass der zum ersten DNA Strang komplementäre zweite DNA Strang entsprechend den Basenpaarungsregeln die Nukleotide aufweist, die zu den Basen des ersten Stranges korrespondieren.

Der Begriff "(molekularer) Marker" ist eine Nukleotidsequenz, die als Bezugs- oder Orientierungspunkt verwendet wird. Ein Marker zum Erkennen eines Rekombinationsereignisses sollte geeignet sein, Unterschiede oder Polymorphismen innerhalb einer pflanzlichen Population zu überwachen. Für Marker finden sich diese Unterschiede auf DNA-Ebene und sind beispielsweise Polynukleotidsequenzunterschiede wie zum Beispiel SSRs (*simple sequence repeats*), RFLPs (*restriction fragment length polymorphisms*)*,* FLPs (*fragment length polymorphisms*) oder SNPs (*single nucleotide polymorphisms*)*.* Die Marker können von genomischen oder exprimierten Nukleinsäuren wie beispielsweise gespleißter RNA, cDNA oder ESTs abgeleitet sein und können sich auch auf Nukleinsäuren beziehen, die als Sonden oder Primer-Paare eingesetzt werden und als solche geeignet sind, ein Sequenzfragment unter Verwendung PCR-basierter Verfahren zu amplifizieren. Marker, welche genetische Polymorphismen zwischen Teilen einer Population betreffen, können mittels etablierter Verfahren aus dem Stand der Technik nachgewiesen werden (An Introduction to Genetic Analysis. 7th Edition, Griffiths, Miller, Suzuki et al., 2000). Dazu gehören z. B.: DNA-Sequenzierung, PCR-basierte, sequenzspezifische Amplifikation, Nachweis von RFLPs, Nachweis von Polynukleotidpolymorphismen mittels Allel-spezifischer Hybridisierung (ASH), Detektion von SSRs, SNPs oder RFLPs. Darüber hinaus sind auch Verfahren zur Detektion von ESTs (expressed sequence tags) und RAPD (randomly amplified polymorphic DNA) bekannt. Je nach Kontext kann der Begriff Marker in der Beschreibung auch eine spezifische Chromosom-Position in dem Genom einer Spezies, wo ein spezifischer Marker (z.B. SNP) gefunden werden kann, meinen. Marker werden in dieser Erfindung auch zum Detektieren von Deletionsereignissen verwendet.

Der Begriff "Nutzpflanze" schließt sowohl wild wachsende Pflanzen als auch Kulturpflanzen ein. Als Nutzpflanzen werden Pflanzen bezeichnet, die in irgendeiner Form vom Menschen in direkter oder indirekter Weise genutzt werden; z.B. als Nahrungsmittel, Genussmittel, Heilmittel, sowie als Holzlieferant, oder auch als Futtermittel für Nutztiere.

Eine "Kulturpflanze" ist im Gegensatz zur Wildpflanze eine vom Menschen angebaute, gepflegte und gezüchtete Pflanze, die als Nutzpflanze oder Zierpflanze Verwendung findet. Genetische Basis der Entstehung von Kulturpflanzen sind Punktmutationen, somatischen Mutationen, Chromosomenmutationen sowie Polyploidisierung. Diese Mutationen liefern die Grundlage für die Selektion. Sie bilden das natürliche oder durch künstliche Hilfsmittel (Steigerung der Mutationsrate, Kreuzungszüchtung, Behandlung mit Colchicin, gentechnische Methoden) erweiterte Ausgangsmaterial einer vom Menschen gelenkten Evolution. Zu den Kulturpflanzen zählen u.a. die Nahrungspflanzen, Industriepflanzen (z.B. Faserpflanzen), Futterpflanzen und Zierpflanzen. Wichtige Merkmale dieser Kulturpflanzen sind u.a. die Größenzunahme der Pflanze, insbesondere der genutzten Organe, Verlust von Bitterstoffen, Schädlingsresistenz, und/oder hoher Nährstoffgehalt.

"Operativ verknüpft" meint verbunden in einem gemeinsamen Nukleinsäuremolekül, in einer Weise, dass die verbundenen Elemente derart zueinander positioniert und orientiert sind, dass eine Transkription des Nukleinsäuremoleküls stattfinden kann. Eine DNA, welche operativ mit einem Promotor verknüpft ist, steht unter der transkriptionellen Kontrolle dieses Promotors.

Eine "Pflanze" im Sinne der Anmeldung kann, sofern nicht anders angegeben, von jeder Spezies aus den dikotyledonen und monokotyledonen Pflanzen sein. Bevorzugt sind Pflanzen in Agrikultur oder Hortikultur oder zur Erzeugung von Bioenergie (Bioethanol, Biogas etc.). Vorzugsweise zeichnen sich die in der vorliegenden Anmeldung gezeigten Pflanzen durch Speicherorgane aus wie Knollen, Wurzeln, Samen, Korn, Früchten, etc. Hierzu zählen beispielhaft *Zea mays, Solanum tuberosum, Triticum aestivum, Triticum durum, Triticum spelta, Helianthus annuus, Secale cereale, Hordeum vulgare, Hordeum bulbosum, Brassica napus, Brassica oleracea, Brassica rapa, Brassica juncacea, Brassica nigra, Glycine max, Gossypium sp., Sorghum bicolor,* Triticale, *Saccharum officinarium, Setaria italica, Oryza sativa, Oryza minuta, Oryza australiensis, Oryza alta, Brachypodium distachyon, Hordeum marinum, Aegilops tauschii, Daucus glochidiatus, Daucus pusillus, Daucus muricatus, Daucus carota, Eucalyptus grandis, Erythranthe guttata, Genlisea aurea, Musa sp., Avena sp., Nicotiana sylvestris, Nicotiana tabacum, Nicotiana tomentosiformis, Solanum lycopersicum, Coffea canephora, Vitis vinifera, Cucumis sativus, Morus notabilis, Crucihimalaya himalaica, Crucihimalaya wallichii, Cardamine flexuosa, Lepidium virginicum, Capsella bursa-pastoris, Olmarabidopsis pumila, Arabis hirsuta, Raphanus sativus, Eruca vesicaria sativa, Citrus sinensis, Jatropha curcas, Populus trichocarpa* oder *Beta vulgaris..* Eine erfindungsgemäße Pflanze ist vorzugsweise eine Pflanze der Gattung *Beta,* insbesondere der Spezies Zuckerrübe (*Beta vulgaris*)*,* sowie der Sub-Spezies *Beta vulgaris* subsp. *vulgaris.*

Pflanzliche "Organe" meinen beispielsweise Blätter, Sprossachse, Stamm, Wurzeln, vegetative Knospen, Meristeme, Embryos, Antheren, Ovula, Samen oder Früchte, insbesondere Samenkörner. Der Begriff "Pflanzenteil" bzw. "Pflanzenteile" beinhaltet, ist jedoch nicht beschränkt auf, die Sprossachse bzw. den Halm, Blätter, Blüten, Blütenstände, Wurzeln, Früchte und Samen sowie die Pollen. Pflanzliche "Teile" meinen ferner einen Zusammenschluss mehrerer Organe, z.B. eine Blüte oder ein Samen, oder einen Teil eines Organs, z.B. einen Querschnitt aus der Sprossachse. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe sowie das Bildungsgewebe, Grundgewebe (das sogenannte Parenchym), Leitgewebe, Festigungsgewebe und das Deckgewebe (die sogenannte Epidermis). Das Gewebe wird durch diese Auflistung jedoch nicht beschränkt. Unter pflanzlichen "Zellen" sind beispielsweise isolierte Zellen mit einer Zellwand oder Aggregate davon oder Protoplasten zu verstehen.

Der Begriff "regulatorische Sequenz" betrifft eine Nukleotidsequenz, welche die Spezifität und/oder die Expressionsstärke beeinflusst, zum Beispiel indem die regulatorische Sequenz eine bestimmte Gewebespezifität vermittelt. Eine solche regulatorische Sequenz kann stromaufwärts des Transkriptionsinitiationspunkts eines Minimalpromotors, aber auch stromabwärts davon wie beispielsweise in einer transkribierten aber nicht translatierten Leader-Sequenz oder innerhalb eines Introns lokalisiert sein.

Ein "Promotor" ist ein nicht-translatierter DNA-Abschnitt, typischerweise stromaufwärts einer kodierenden Region, welche die Bindestelle für die RNA-Polymerase beinhaltet und die Transkription der DNA initiiert. Ein Promotor enthält zudem andere Elemente, die als Regulatorgen der Genexpression fungieren (z.B. cis-regulatorischen Elemente). Ein "Kern-oder Minimalpromotor" ist ein Promoter, der zumindest die Grundelemente, welche für die Transkriptionsinitiation gebraucht werden, aufweist (z.B. TATA-Box und/oder Initiator).

Eine "Transgene Pflanze" bezieht sich auf eine Pflanze, in deren Genom mindestens ein Polynukleotid, vorzugsweise ein heterologes Polynukleotid, integriert ist. Bevorzugt ist das Polynukleotid stabil integriert, was bedeutet, dass das integrierte Polynukleotid in der Pflanze stabil erhalten bleibt, exprimiert wird und auch stabil an die Nachkommen vererbt werden kann. Das stabile Einbringen eines Polynukleotids in das Genom einer Pflanze schließt auch die Integration in das Genom einer Pflanze der vorhergehenden Parentalgeneration mit ein, wobei das Polynukleotid stabil weitervererbt werden kann. Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

Ausgestaltungen und Ausführungsformen der vorliegenden Erfindung werden in exemplarischer Weise mit Bezug auf die angehängten Abbildungen und Sequenzen beschrieben:

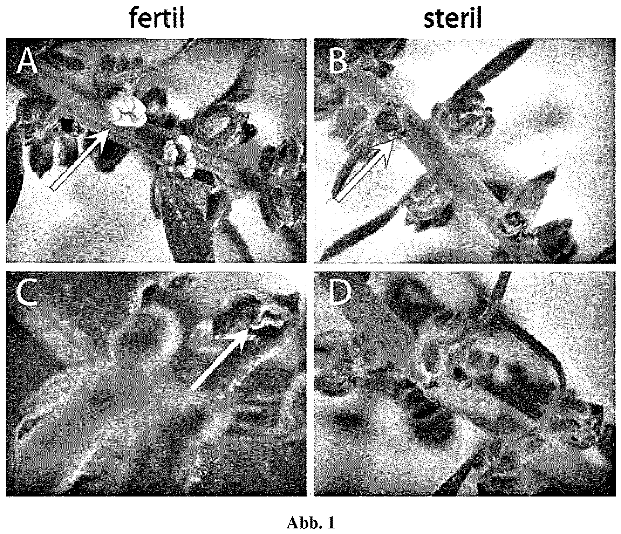

**Abb. 1****: A, C)** Blüten von fertilen Zuckerrüben (*Beta vulgaris* subsp. *vulgaris*) und **B, D)** Blüten von männlich sterilen Zuckerrüben, deren Phänotyp auf den Donor C311 [2043_K5] zurückgeht. **A, B)** Geschlossene Blüten, deren Sepalen und Petalen manuell entfernt wurden. Deutlich zu sehen sind die hellen (gelben), vitalen Antheren des fertilen Genotyps **(A).** Im Gegensatz dazu sind die Antheren der sterilen Genotypen deutlich dunkel (braun) verfärbt. Während der Blütenreifung öffnen sich die Antheren fertiler Genotypen und entlassen Pollen **(C),** während die Antheren steriler Genotypen nicht weiter reifen und keinen Pollen enthalten.

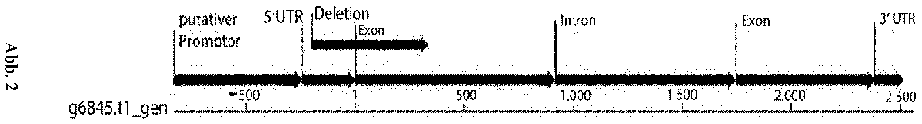

**Abb. 2****:** In RefBeet 1.2 annotiertes Genmodel von BvCYP*gst* (g6845.t1) im Referenzgenotyp KWS2320. Das Protein mit einer Länge von 517 Aminosäuren wird von zwei Exons mit einer Gesamtlänge von 1554 bp kodiert. Genotypen, die einen männlich sterilen Phänotyp ausprägen zeigen eine Deletion von 533 bp, die Teile der 5' UTR und des ersten Exons des Gens umfasst. Eine korrekte Transkription der mRNA und Translation eines funktionsfähigen Proteins ist damit ausgeschlossen.

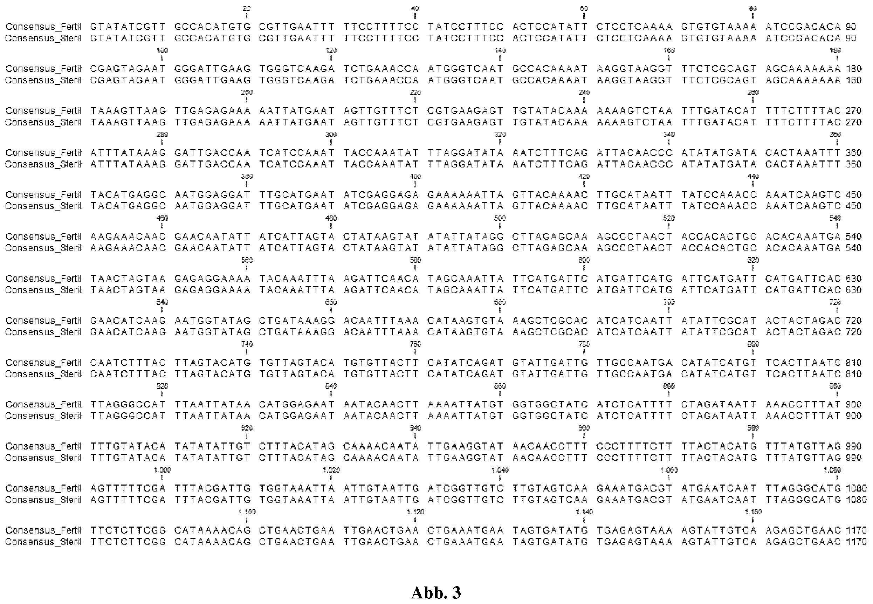

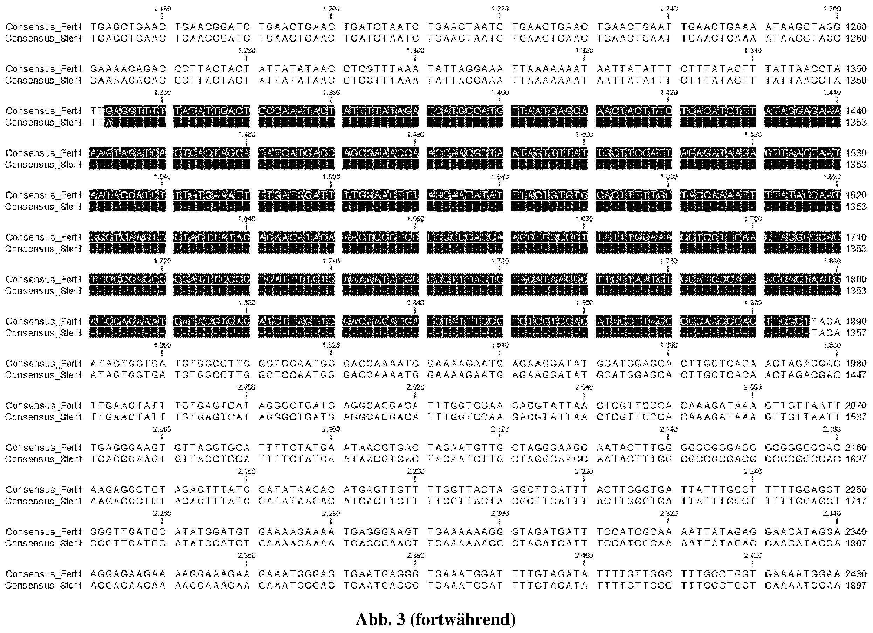

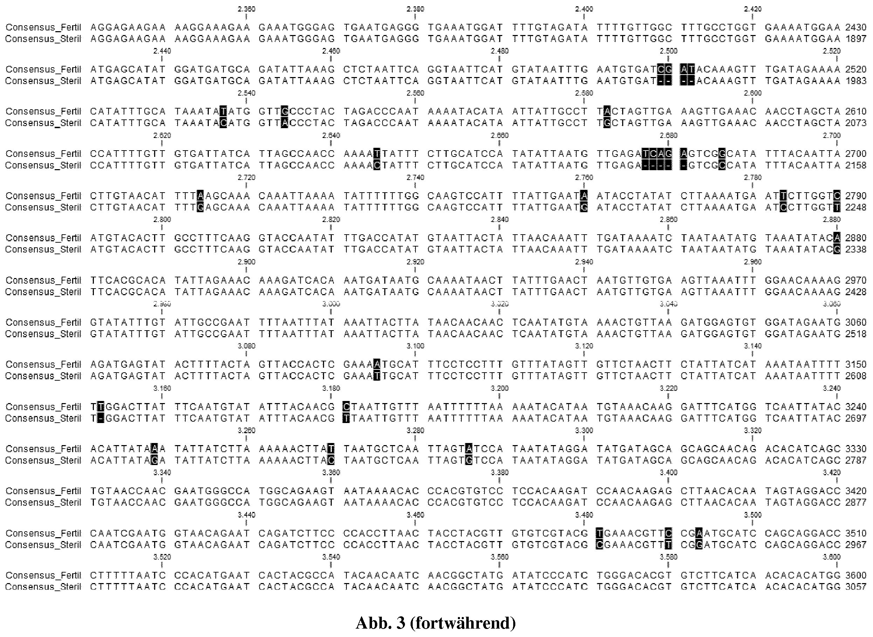

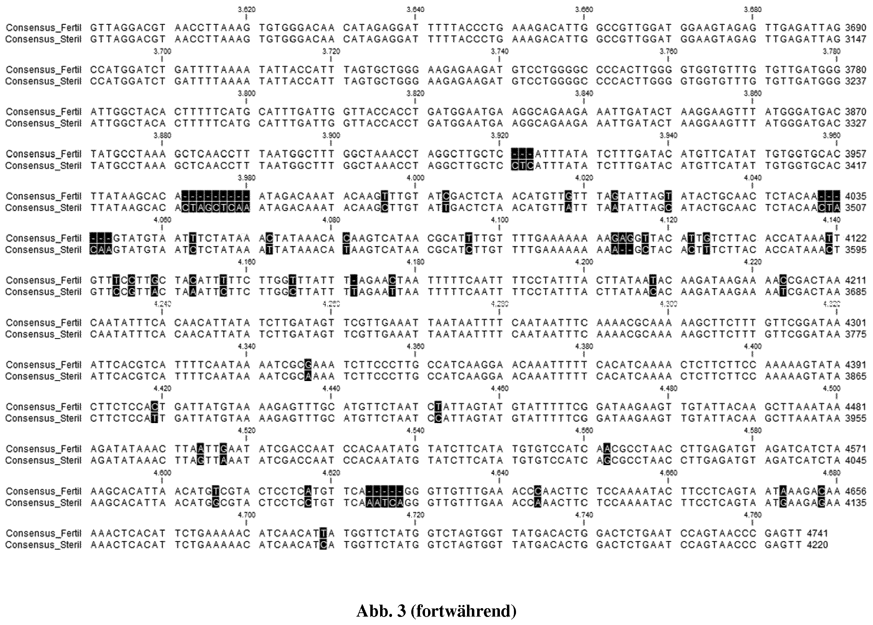

**Abb. 3****:** Alignment eines 4721 bp genomischen DNA Fragments, das das Zuckerrüben-Genmodel von BvCYP*gst* (g6845.t1) kodiert, von sterilen und fertilen Genotypen. Die Sequenz der sterilen Genotypen weist eine 533 bp Deletion auf.

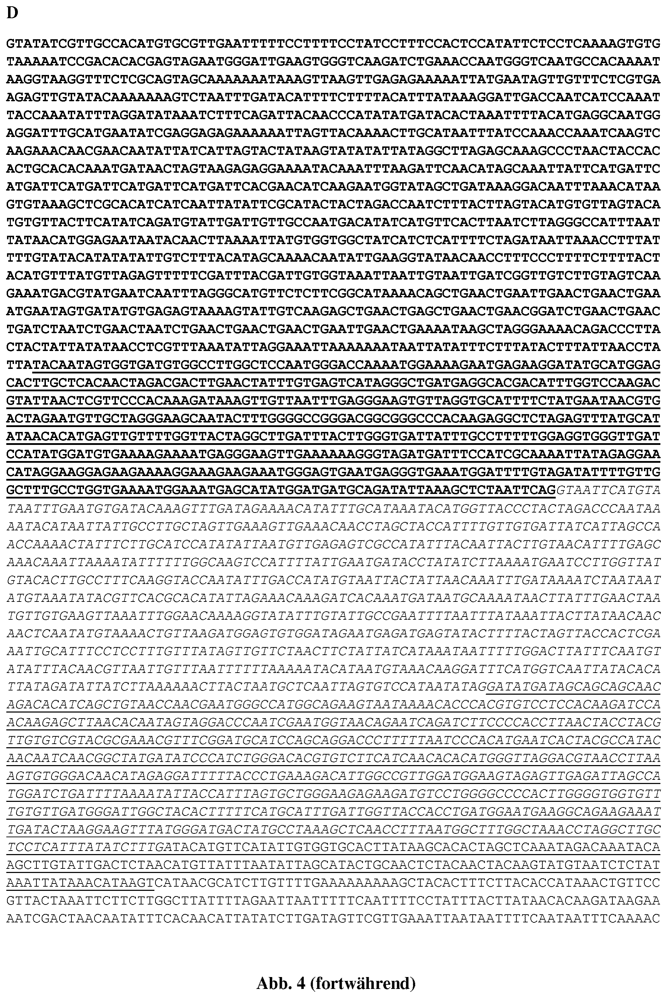

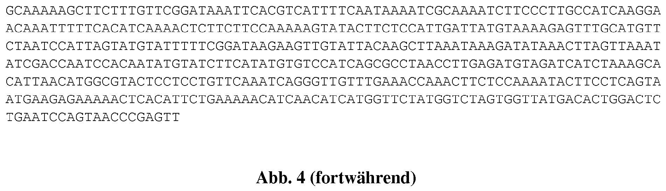

**Abb.** 4: Sequenzanalyse des 4721 bp genomischen DNA Fragments, das das Zuckerrüben-Gen BvCYP*gst* (g6845.t1) kodiert, von sterilen und fertilen Genotypen. **A)** Dargestellt ist die genomische DNA-Sequenz des CYP*gst* Gens aus *Beta vulgaris* subsp. *vulgaris* inklusive der putativen Promotorregion sowie der 5'UTR und 3'UTR. Die putative Promotorregion ist in "fett" dargestellt, die 5'UTR sowie die 3'UTR sind unterstrichen, Exon 1 ist "fett" und unterstrichen, Exon 2 ist "kursiv" und unterstrichen und das Intron ist in "kursiv" dargestellt. Diese Sequenz entspricht der in SEQ ID Nr. 1 hinterlegten Sequenz. Die funktionellen Bereiche des Gens sind wie folgt lokalisiert: Putativer Promotor 1..1518; 5'UTR 1519..1761; transkribierter Bereich 1519..4275; Exon 1762..2679; Intron 2680..3506; Exon 3507..4142; 3'UTR 4143..4275. **B)** Dargestellt ist die cDNA-Sequenz des CYP*gst* Gens aus *Beta vulgaris* subsp. *vulgaris* inklusive der 5'UTR und 3'UTR. Die 5'UTR sowie die 3'UTR sind unterstrichen, Exon 1 ist "fett" und unterstrichen und Exon 2 ist "kursiv" und unterstrichen dargestellt. Diese Sequenz entspricht der in SEQ ID Nr. 2 hinterlegten Sequenz. Die funktionellen Bereiche der cDNA sind wie folgt lokalisiert: 5'UTR 1..243; Exon 244..1161; Exon 1162..1797; 3'UTR 1798..1930. **C)** Dargestellt ist die Aminosäuresequenz des CYP*gst* Gens aus *Beta vulgaris* subsp. *vulgaris.* Diese Sequenz entspricht der in SEQ ID Nr. 3 hinterlegten Sequenz. **D)** Dargestellt ist die genomischen DNA-Sequenz des mutierten CYPgst Gens aus *Beta vulgaris* subsp. *vulgaris* inklusive der putativen Promotorregion sowie der 3'UTR. Die putative Promotorregion ist in "fett" dargestellt, die 3'UTR ist unterstrichen, das verkürzte Exon 1 ist "fett" und unterstrichen, Exon 2 ist "kursiv" und unterstrichen und das Intron ist in "kursiv" dargestellt. Diese Sequenz entspricht der in SEQ ID Nr. 8 hinterlegten Sequenz. Die funktionellen Bereiche des mutierten CYP*gst* Gens sind wie folgt lokalisiert: Putative Promoter 1..1353; transkribierter Bereich 1354..3542; trunkiertes Exon 1354..1938; Intron 1939..2755; Exon 2756..3394; 3'UTR 3395..3542.

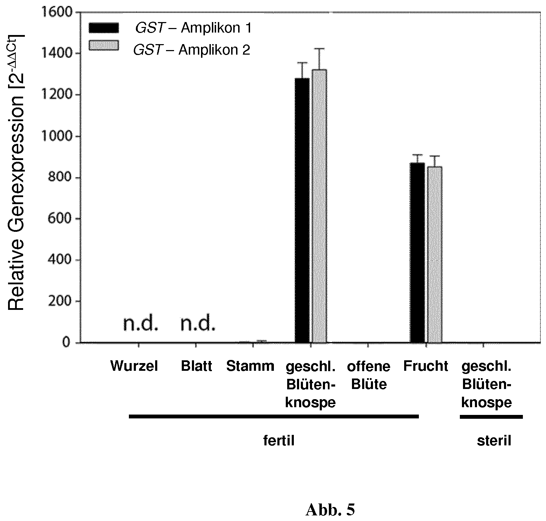

**Abb. 5****:** Expressionsanalyse des Gens *BvCYPgst* (*GST,* g6845.t1) mittels qRT-PCR. RNA wurde aus verschiedenen Geweben von fertilen Pflanzen gewonnen und die Expression des *GST-*Gens im Vergleich zur Expression des Gens g4645.t1 dargestellt. n.d., keine Expression nachweisbar. *GST* wird im gezeigten Experiment in geschlossenen Blüten am stärksten exprimiert. Als Vergleich ist in geschlossenen Blüten der sterilen Genotypen keine Expression des *GST* nachweisbar.

### AUSFÜHRLICHE BESCHREIBUNG DER ERFINDUNG

Die vorliegende Erfindung wird definiert durch die Merkmale der unabhängigen Ansprüche.

In einem Aspekt betrifft die Erfindung eine Pflanze der Art *Beta vulgaris,* die einen rezessiven, kernkodierten männlich sterilen Phänotyp zeigt, dadurch gekennzeichnet, dass der Phänotyp mit einer Mutation im endogenen Cytochrom P450 Oxidase Gen korreliert, dadurch gekennzeichnet, dass es sich bei dem nicht-mutierten Cytochrom P450 Oxidase Gen um ein Gen handelt ausgewählt aus der Gruppe bestehend aus:
(a) einer Nukleotidsequenz, die die in SEQ ID Nr.: 1 oder SEQ ID Nr.: 2 gezeigte Nukleotidsequenz oder ein funktionelles Fragment davon aufweist;
(b) einer Nukleotidsequenz, die die in SEQ ID Nr.: 3 gezeigte Aminosäuresequenz kodiert;
(c) einer Nukleotidsequenz, die in der Lage ist, an eine Nukleotidsequenz komplementär zu einer Nukleotidsequenz gemäß (a) oder (b) unter stringenten Bedingungen zu hybridisieren; und
(d) einer Nukleotidsequenz, die eine Aminosäuresequenz kodiert, die eine Sequenzidentität von mindestens 80% über die gesamte Aminosäuresequenz gemäß SEQ ID Nr.: 3 aufweist,

wobei es sich bei der Mutation um eine Deletion, Addition, Insertion oder Substitution in der kodierenden Nukleotidsequenz des Cytochrom P450 Oxidase Gens, in einem Splicing-Signal oder in einer regulatorischen Sequenz, vorzugsweise der Promotor-sequenz, des Cytochrom P450 Oxidase Gens handelt, und
wobei die besagte Mutation die Transkription und/oder Translation eines funktionellen Cytochrom-P450-Oxidase-Proteins reduziert oder verhindert.

Gemäß einer bevorzugten Ausführungsform der vorliegenden Erfindung ist die besagte Pflanze homozygot für die Mutation und männlich steril, wobei in der sterilen Pflanze die Ausbildung von funktionellem Pollen unterbunden ist.

Bevorzugt handelt es sich bei der Mutation um eine Deletion zwischen den Nukleotidpositionen 1560 und 2095 der SEQ ID Nr.: 1.

Bevorzugt kann die besagte Deletion durch Abwesenheit einer oder beider der Markerloci sle5983d14 (Amplifikationsprodukt der Primer mit SEQ ID Nr.: 4 und 5) und sle5983d17 (Amplifikationsprodukt der Primer mit SEQ ID Nr.: 6 und 7) und durch Anwesenheit eines ubiquitären Markers nachgewiesen werden.

Gemäß einer bevorzugten Ausführungsform der vorliegenden Erfindung ist bei der erfindungsgemäßen Pflanze das Gen in einem Segment auf Chromosom 1 zwischen den Markerloci sxn2151s01 und sle3305s02 lokalisiert, wobei die sxn2151s01-Markersequenz dargestellt in SEQ ID Nr.: 24 und die sle3305s02-Markersequenz dargestellt in SEQ ID Nr.: 26 das Vorhandensein des Locus, der die kernkodierte männliche Sterilität verursacht, anzeigen und die sxn2151s01-Markersequenz dargestellt in SEQ ID Nr.: 25 und die sle3305s02-Markersequenz dargestellt in SEQ ID Nr.: 27 die Referenzsequenz anzeigen.

In einem weiteren Aspekt betrifft die vorliegende Erfindung ein rekombinantes DNA-Molekül umfassend eine Nukleotidsequenz, die eine shRNA *(small hairpin RNA),* siRNA *(small interfering RNA),* Antisense-RNA, Sense-RNA oder doppelsträngige RNA kodiert, die nach Expression in einer Pflanzenzelle oder nach Einbringen in eine Pflanzenzelle zur Reduktion oder Inhibierung der Expression des besagten funktionellen (nicht-mutierten) Cytochrom P450 Oxidase Gens, wie oben definiert, führt.

In einem weiteren Aspekt betrifft die vorliegende Erfindung eine Pflanzenzelle, die das besagte rekombinante DNA-Molekül enthält.

In einem weiteren Aspekt betrifft die vorliegende Erfindung ein Verfahren zur Herstellung einer männlich sterilen Pflanze der Art *Beta vulgaris,* dadurch gekennzeichnet, dass die Expression des Cytochrom P450 Oxidase Gens gemäß einer der oben genannten Nukleotidsequenzen (a), (b), (c), oder (d) reduziert oder inhibiert wird, wobei das Verfahren einen Schritt des Einbringens des rekombinanten DNA-Moleküls nach Anspruch 8 umfasst, wobei besagter Schritt des Verfahrens eine Agrobacterium-Transformation, ein T-DNA-Tagging, oder eine Mutagenese mittels TILLING umfasst, oder einen Schritt der gezielten Mutagenese der Nukleotidsequenzen (a), (b), (c), oder (d) umfasst, infolgedessen die Expression des Gens beispielsweise durch RNAi oder Co-Suppression oder aufgrund der eingebrachten Mutation reduziert oder inhibiert wird.

In einem weiteren Aspekt betrifft die vorliegende Erfindung eine erfindungsgemäße Pflanze enthaltend eine Pflanzenzelle, die das besagte rekombinante DNA-Molekül enthält und/oder die erhältlich ist durch eines der vorgenannten Verfahren zur Herstellung einer männlich sterilen Pflanze der Art *Beta vulgaris.*

In einem weiteren Aspekt betrifft die vorliegende Erfindung ein Verfahren zur Restauration der Fertilität einer erfindungsgemäßen Pflanze, umfassend das Einbringen eines funktionellen (nicht-mutierten) Cytochrom P450 Oxidase Gens in die Pflanze durch Transformation mittels einer rekombinanten DNA, die eine nicht-mutierte Nukleotidsequenz mit einer kodierenden Sequenz gemäß der oben genannten Nucleotidsequenzen (a) - (d) umfasst, die operativ mit einem heterologen Promoter verknüpft ist, der vorzugsweise in der Lage ist, die Expression der Nukleotidsequenz spezifisch in geschlossenen Blüten und/oder Früchten zu steuern.

In einem weiteren Aspekt betrifft die vorliegende Erfindung ein Organ, Pflanzenteil, Gewebe oder eine Zelle der erfindungsgemäßen Pflanze oder einen Samen oder Nachkommen der erfindungsgemäßen Pflanze, wobei der Samen oder die Nachkommen die erfindungsgemäß definierte Mutation und/oder das erfindungsgemäße rekombinante DNA-Molekül aufweist bzw. aufweisen.

In einem weiteren Aspekt betrifft die vorliegende Erfindung ein Verfahren zur Identifizierung einer erfindungsgemäßen Pflanze durch Nachweis der Mutation in dem Cytochrom P450 Oxidase Gen bzw. eines Markers, der mit der Mutation gekoppelt ist.

In einem weiteren Aspekt betrifft die vorliegende Erfindung die Verwendung eines Nukleinsäuremoleküls von mindestens 15 Nukleotiden Länge, das spezifisch an eine der oben definierten Nukleotidsequenzen (a) - (d) hybridisiert, und/oder eines Oligonukleotids, vorzugsweise mit einer Länge von maximal 50 Nukleotiden, das eine der folgenden Nukleotidsequenzen aufweist:
(i) SEQ ID Nr.: 4, 6 oder einem Komplement davon, oder
(ii) SEQ ID Nr.: 5, 7 oder einem Komplement davon,
zur Identifizierung einer erfindungsgemäßen Pflanze.

Die vorliegende Erfindung stellt eine Pflanze zur Verfügung, die durch Mutation in einem DNA-Segment des Kerngenoms, das ein Cytochrom P450 Oxidase (CYP*gst*) Gen umfasst, einen kernkodierten männlich sterilen Phänotyp aufweist. Dieser ist dadurch ausgezeichnet, dass die Mutation durch eine rezessive Merkmalsausprägung erhalten wird und die Pflanze somit zur Vereinfachung von arbeitsaufwendigen Züchtungsprogrammen eingesetzt werden kann. Die Identifizierung des für diese Merkmalsausprägung verantwortlichen Gens erfolgte in der Zuckerrübe (*Beta vulgaris* subsp. *vulgaris*) wie in den Beispielen 1 und 2 nebst Abbildungen 1 bis 5 beschrieben. Das betreffende Gen wurde aufgrund seiner durch Sequenzanalyse bestimmten strukturellen Merkmale als ein Mitglied der Cytochom P450 Oxidasen (CYP) klassifiziert und aufgrund des für seine Mutante beobachteten Phänotyp der kerngenetischen männlichen Sterilität mit dem Suffix *"gst"* versehen. Da das Gen in Zuckerrübe identifiziert wurde, wird ferner das Präfix "*Bv*" verwendet wenn konkret auf das in den Beispielen beschriebene Gen Bezug genommen wird.

Im Allgemeinen betrifft die vorliegende Erfindung eine Pflanze, insbesondere Kultur- bzw. Nutzpflanze, die einen rezessiven, kernkodierten männlich sterilen Phänotyp zeigt, dadurch gekennzeichnet, dass der Phänotyp mit einer Mutation, welche von dem endogenen Cytochrom P450 Oxidase (CYP*gst*) Gen umfasst wird, oder mit der Abwesenheit bzw. im Vergleich zu einer entsprechenden (männlich fertilen) Wildtyppflanze geringen Gehalt bzw. Aktivität eines funktionsfähigen CYP*gst* Proteins, das durch das Wildtypgen von CYP*gst* kodiert wird, korreliert, dadurch gekennzeichnet, dass es sich bei dem nicht-mutierten CYP*gst* Gen um das Gen *Bv*CYP*gst* aus *Beta vulgaris* handelt, das eine der in SEQ ID Nr.: 1 oder 2 gezeigte Nukleotidsequenz umfasst bzw. die in SEQ ID Nr.: 3 gezeigte Aminosäuresequenz kodiert, oder um dessen Homolog, Analog, oder Ortholog. Wie vorstehend beschrieben und in den Beispielen erläutert können durch klassische bioinformatische Ansätze (Datenbankrecherche und Computerprogramme für das Screening nach homologen Sequenzen) weitere CYP*gst* Proteine bzw. deren kodierende Gene, d.h. Homologe, Analoge und Orthologe in Pflanzen identifiziert werden, wobei davon auszugehen ist, dass eine Mutation den gleichen Phänotyp hervorrufen wird, wie in der Zuckerrübe beobachtet. Somit ist auch eine Pflanze offenbart, die dadurch gekennzeichnet ist, dass es sich bei dem nicht-mutierten CYP*gst* Gen um das Gen *St*CYP*gst* aus *Solanum tuberosum* handelt, das vorzugsweise eine der in SEQ ID Nr.: 12 oder 13 gezeigte Nukleotidsequenz umfasst bzw. die in SEQ ID Nr.: 14 gezeigte Aminosäuresequenz kodiert, oder um dessen Homolog, Analog oder Ortholog; oder dass es sich bei dem nicht-mutierten CYPgst Gen um das Gen *Zm*CYP*gst* aus *Zea mays* handelt, das vorzugsweise eine der in SEQ ID Nr.: 9 oder 10 gezeigte Nukleotidsequenz umfasst bzw. die in SEQ ID Nr.: 11 gezeigte Aminosäuresequenz kodiert, oder um dessen Homolog, Analog oder Ortholog.

Der Begriff Homolog(e) bedeutet dabei, dass die betreffenden Gene (aus zwei verschieden Pflanzenarten) im Wesentlichen die gleichen Funktion und einen gemeinsamen Vorläufer haben, und sich daher typischerweise eine signifikante Identität in deren Nukleinsäure- bzw. kodierten Aminosäuresequenz zeigt. Es gibt aber auch viele Gene, die zueinander homolog sind, ohne dass Protein-Sequenzen ein sinnvolles paarweises Alignment ergeben. Im Gegensatz dazu beschreibt der Begriff Analog(e) Gene bzw. Proteine, die (ebenfalls) eine identische oder ähnliche Funktion haben, aber nicht aus derselben Struktur entstanden sind, d.h. keine gemeinsamen Vorläufer haben. In diesem Fall lässt sich oftmals keine signifikante Identität in deren Nukleinsäure- bzw. kodierten Aminosäuresequenz feststellen oder bestenfalls in bestimmten funktionellen Domänen.

Homologe werden im Kontext der Genom-Sequenzierung zur Annotation feiner klassifiziert. Dazu wurden die Begriffe Orthologie und Paralogie eingeführt. Orthologe sind Gene, die über ein Speziationsereignis verbunden sind. Paraloge sind Gene, die über ein Duplikationsereignis verbunden.

Ein Gen ist grundsätzlich dann ein Homolog bzw. Analog oder Ortholog im Sinne der vorliegenden Erfindung wenn es in der Lage ist, den männlich sterilen Phänotyp bei dem Referenzgen CYP*gst* in Zuckerrübe (*BvCYPgst*) zu komplementieren und/oder eine zielgerichtete Mutation in dem betreffenden Gen bzw. Veränderungen der biologischen Aktivität des durch das Homolog oder Analog kodierten Genprodukts ein männlich steriler Phänotyp in der Pflanze, aus der das Gen stammt hervorgerufen wird. Dementsprechend kann das betreffende Homolog bzw. Analog zum in den Beispielen illustrierten CYP*gst* Gen der vorliegenden Erfindung vorzugsweise dadurch gekennzeichnet werden, dass es in der Lage ist den männlich sterilen Phänotyp, der für die CYP*gst* Mutante der Zuckerrübe beobachtet wird zu komplementieren, d.h. den fertilen Phänotyp zu restaurieren. Zusätzlich oder alternativ kann das CYP*gst* Homolog bzw. Analog vorzugsweise dadurch gekennzeichnet werden, dass durch eine Inhibierung dessen Expression oder der biologischen Aktivität des durch das Homolog oder Analog kodierten Genprodukts ein männlich steriler Phänotyp hervorgerufen wird. Vorzugsweise weist der männlich sterile Phänotyp die beispielhaft für die CYP*gst* Mutante aus Zuckerrübe, insbesondere in den Beispielen beschriebenen Eigenschaften auf; siehe auch die vorstehend beschriebenen Ausführungsformen.

Entsprechende Techniken und Verfahren zu Komplementationsgenetik sind dem Fachmann aus dem Stand der Technik bekannt; siehe beispielsweise Napoli et al., Plant Physiology 120 (1999), 615-622, die eine Mutation in einer Inzuchtlinie von Petunie beschreibt, die unter anderem ein männlich sterilen Phänotyp aufweist, der durch transgene Komplementation mit einer funktionellen Chalcon Synthase A cDNA aufgehoben wurde und somit festgestellt werden konnte, dass das Chalcon Synthase Gen A wesentlich für den männlich sterilen Phänotyp ist bzw. der Phänotyp der männlichen Sterilität durch eine Mutation in diesem Gen hervorgerufen wurde.

In Jeong et al., J. Exp. Bot. 65 (2014), 6693-6709, wurde durch Komplementation und mittels transgener Expression verschiedener Kandidatengene die männliche Sterilität in der sogenannten *ms10³⁵* Mutante von Tomate komplementiert und aufgehoben. Verfahren zur Herstellung männlicher Sterilität in transgenen Pflanzen durch Inhibierung eines Zielgens, in diesem Fall CYP*gst* sind dem Fachmann ebenfalls bekannt; siehe beispielsweise die internationale Anmeldung WO 1996/017945 sowie die nachstehend beschrieben Ausführungsformen.

Somit ist in einer Ausführungsform der vorliegenden Erfindung eine Pflanze, die einen rezessiven, kernkodierten, männlich sterilen Phänotyp zeigt, dadurch gekennzeichnet, dass der Phänotyp durch eine Mutation, welche von dem endogenen Cytochrom P450 Oxidase (CYP*gst*) Gen umfasst wird, hervorgerufen wird. Die erfindungsgemäße Pflanze kann aber auch durch die Abwesenheit bzw. im Vergleich zu einer entsprechenden männlich fertilen Wildtyppflanze geringen Gehalt bzw. Aktivität eines funktionsfähigen CYP*gst* Proteins, das durch das Wildtypgen von CYP*gst* kodiert wird, gekennzeichnet sein. Eine genomische Sequenz des mutierten Gens, welches nicht mehr translatiert werden kann, ist in SEQ ID Nr. 8 offenbart. Insbesondere betrifft die Anmeldung eine Pflanze, die einer Kultur- und Nutzpflanze zuzuordnen ist.

Im Stand der Technik (Morant et al., The Plant Cell, 19 (2007), 1473-1487) ist beschrieben, dass eine Ausschaltung des CYP703 Gens in *Arabidopsis thaliana* (*CYP703A2*) zu einer reduzierten Pollenbildung und somit zu einer partiellen männlichen Sterilität führt. Dies ist darauf zurückzuführen, dass das Sporopollenin, als Hauptbestandteil der Exin-Schicht von Pollen, abwesend oder strukturell verändert war. Obwohl es plausibel klingt, dass das CYP*gst* Gen eine andere Funktion übernimmt, da eine Mutation zu einer kernkodierten, rezessiven, männlichen Sterilität führt und es nicht zu einer Ausbildung von Pollen kommt, soll nicht ausgeschlossen werden, dass das CYP*gst* Gen und das CYP703 Gen aus *Arabidopsis thaliana* zur gleichen Genfamilie gehören und somit die gleiche oder zumindest ähnliche Funktion in der Synthese des Sporopollenins erfüllen. Ohne an eine bestimmte Theorie gebunden zu sein, klingt es jedoch plausibel, dass in Kulturpflanzen, die durch jahrelange gezielte Selektion und Kreuzungen hinsichtlich u.a. Ertrag, Schädlingsresistenz, Toleranz gegen abiotische Stressfaktoren, sowie Gehalts pflanzlicher Inhaltsstoffe optimiert wurden, die Fähigkeit zur Kompensation des fehlenden Sporopollenins abhandengekommen ist und dass somit die Abwesenheit von Sporopollenin dazu führt, dass die Ausbildung von Pollen unterbunden wird und die Pflanze somit männlich steril ist.

Da in Morant *et al.* (2007) beschrieben ist, dass das CYP703A2 Gen bzw. entsprechende Knockout Linien von Arabidopsis lediglich ein partiell männlich sterilen Phänotyp aufweisen, und sich ein solcher Phänotyp nicht für die Hybridzüchtung eignet, ist in einer Ausführungsform das CYP703A2 Gen aus *Arabidopsis thaliana* bzw. die in Morant *et al.* beschriebene Mutanten, insbesondere mit den in Figur 1 gezeigten Sequenzen von der vorliegenden Erfindung ausgenommen.

Pflanzen besitzen als Eukaryoten zwei oder mehr Kopien ihrer genetischen Information pro Zelle. Jedes Gen wird in der Regel durch zwei Allele, die im homozygoten Zustand identisch bzw. im heterozygoten Zustand unterschiedlich sein können, repräsentiert. Der Phänotyp der erfindungsgemäßen Pflanze wird durch eine Mutation im Kerngenom verursacht und erhalten wird diese durch eine rezessive Merkmalsaufprägung. Dementsprechend ist die Pflanze männlich fertil wenn die Mutation heterozygot vorliegt und männlich steril wenn die Mutation homozygot vorliegt.

In einer sterilen Pflanze ist die Ausbildung von funktionellem Pollen unterbunden, bevorzugt vollständig unterbunden, wobei im Zusammenhang mit der vorliegenden Erfindung der Begriff "unterbunden" bedeutet, dass in einer Pflanze, die homozygot für die Mutation im *CYPgst* Locus und männlich steril ist, die Ausbildung des Pollens zu 95%, vorzugsweise 96%, mehr bevorzugt 97%, besonders bevorzugt 98%, und insbesondere bevorzugt zu 99% nicht stattfindet, während "vollständig unterbunden" meint, dass die Ausbildung des Pollens zu mehr als 99%, vorzugsweise zu 100% unterbunden ist. Vorzugsweise bedeutet in diesem Zusammenhang "unterbunden", dass bei dem Versuch der Kreuzung einer solchen Pflanze als männlicher Elter mit einer entsprechenden Wildtyp-Pflanze im Wesentlichen keine Saatproduktion erfolgt und/oder keine Nachkommen erzeugt werden.

Im Fall von *Beta vulgaris* subsp. *vulgaris* wird dies anhand Abb. 1 deutlich. In geschlossenen Blüten, deren Sepalen und Petalen manuell entfernt wurden, erkennt man die hellen (gelben), vitalen Antheren des fertilen Genotyps (A). Im Gegensatz dazu sind die Antheren der sterilen Genotypen deutlich dunkel (braun) verfärbt (B). Während der Blütereifung öffnen sich die Antheren der fertilen Genotypen und entlassen Pollen (C), während die Antheren steriler Genotypen nicht weiter reifen und keinen Pollen entlassen (D).

In *Arabidopsis thaliana* katalysiert das CYP703 Protein die Umwandlung von mittelkettigen gesättigten Fettsäuren in die entsprechenden einfach-hydroxylierten Fettsäuren, mit einer bevorzugten Hydroxylierung von Laurinsäure an der C7 Position. Ohne an eine bestimmt Theorie gebunden zu sein, klingt es plausibel, dass das *CYPgst* Protein zwar nicht die gleichen, aber eine ähnliche Funktion erfüllt wie das CYP703 Protein aus *Arabidopsis thaliana,* da eine Ausschaltung beider Gene Einfluss auf die Ausbildung von Pollen hat. Somit kann *CYPgst* eine Funktion in der Synthese des Sporopollenins, dem Hauptbestandteil der Exin-Schicht vitaler Pollen zugeschrieben werden.

Das CYP*gst* Protein könnte daher eine Funktion in der Synthese des Sporopollenins aufweisen und die Umwandlung mittelkettiger gesättigter Fettsäuren in die entsprechenden einfach-hydroxylierten Fettsäuren katalysieren, bevorzugt die Hydroxylierung von Laurinsäure an der C7 Position.

Anhand von Transkriptionsanalysen (Beispiel 2) wurde gezeigt, dass in fertilen Genotypen von *Beta vulgaris* subsp. *vulgaris* das CYP*gst* Gen in geschlossenen Blüten und Früchten exprimiert wurde und dass in Wurzeln und Blättern keine Expression nachweisbar war (Abb. 5). Dementsprechend offenbart die Anmeldung eine vorstehend beschriebene Pflanze, wobei das CYPgst Gen mindestens in geschlossenen Blüten und Früchten exprimiert wird, vorzugsweise spezifisch in geschlossenen Blüten und Früchten.

In einer Ausführungsform verhindert die Mutation die Transkription und/oder Translation eines funktionsfähigen Proteins in der erfindungsgemäßen Pflanze, wobei es sich vorzugsweise bei der Mutation um eine Deletion, Addition, Insertion oder Substitution in der kodierenden Nukleotidsequenz des CYP*gst* Gens, einem Splicing-Signal oder in einer regulatorischen Sequenz, vorzugsweise der Promotorsequenz, des CYP*gst* Gens handelt.

Offenbart wird eine Deletion von mindestens 500-600 bp, die den kodierenden Bereich bzw. den Promotorbereich des CYP*gst* Gens betrifft. Die Deletion kann aber auch eine Länge von mindestens 20, 30 oder 50 konsekutiven Basenpaaren, von mindestens 100, 150, 200 oder 250 konsekutiven Basenpaaren oder bevorzugt von mindestens 300, 400 oder 500 konsekutiven Basenpaaren aufweisen. Bei der Addition handelt es sich vorzugsweise um eine Insertion von einem Nukleotid oder mehreren Nukleotiden in die genomische Sequenz, vorzugsweise in die kodierende Gensequenz, die zu einer Leserasterverschiebung führt. Bei der Substitution handelt es sich vorzugsweise um eine Punktmutation in der genomischen Sequenz, vorzugsweise in der kodierenden Gensequenz, die Stopcodons oder Splicing-Fehler erzeugt.

Durch vergleichende Sequenzierung genomischer DNA-Fragmente, die das CYP*gst* Gen und die putative Promotorregion aus sowohl männlich sterilen als auch männlich fertilen *Beta vulgaris* subsp. *vulgaris* Pflanzen umfassen, ging hervor, dass eine Deletion von 533 bp verantwortlich für den männlich sterilen Phänotyp ist (siehe Beispiel 2 und Abb. 3) und die Deletion zwischen den Nukleotidpositionen 1560 und 2095 der SEQ ID Nr. 1 liegt.

Somit handelt es sich in einer weiter bevorzugten Ausführungsform um eine Deletion von 533 bp, die Teile der 5'UTR und des ersten Exons des *CYPgst* Gens aus *Beta vulgaris* subsp. *vulgaris* umfasst; siehe Abb. 3. Das funktionelle Gen *Bv*CYP*gst* umfasst zwei Exons mit einer Gesamtlänge von 1554 bp. Ein in RefBeet 1.2 annotiertes Genmodel ist in Abb. 2 gezeigt und die genomische DNA-Sequenz von CYPgst mit der Deletion, die zu einem verkürztem Exon 1 führt, ist in SEQ ID Nr. 8 aufgeführt. Mögliche Punktmutationen, die zu einem vorzeitigen Transkriptionsabbruch des CYP*gst* Gens aus *Beta vulgaris* subsp. *vulgaris* führen beziehungsweise ein gestörtes Splicing verursachen könnten, sind in Tabelle 1 aufgelistet, wobei diese vorzugsweise zwischen den Nukleotidpositionen 1560 und 2095 der SEQ ID Nr. 1 liegen.

Wie in dem Beispiel 1 der vorliegenden Erfindung gezeigt, konnten im Zuge der Feinkartierung eng-flankierende Marker des CYP*gst* Gens identifiziert werden und somit die Position des CYP*gst* Gens im Genom von *Beta vulgaris* subsp. *vulgaris* ermittelt werden. Dies wiederum stellte die Grundlage für die Entwicklung von genetischen Markern, mit deren Hilfe die Deletion im CYP*gst* Gen nachgewiesen werden konnte.

Dementsprechend ist in einer Ausführungsform der vorliegenden Erfindung die Pflanze dadurch gekennzeichnet, dass in Zuckerrübe (*Beta vulgaris* subsp. *vulgaris*) die Deletion durch Abwesenheit einer oder beider der Markerloci sle5983d14 (Amplifikationsprodukt der Primer mit SEQ ID Nr.: 4 und 5) und sle5983d17 (Amplifikationsprodukt der Primer mit SEQ ID Nr.: 6 und 7) und durch Anwesenheit eines ubiquitären Markers nachgewiesen werden kann. Der ubiquitäre Marker bestätigt hierbei die hinreichende Qualität der DNA-Extraktion.

Des Weiteren ist in einer Ausführungsform das Gen aus *Beta vulgaris* subsp. *vulgaris* (Zuckerrübe) in einem Segment auf Chromosom 1 zwischen den Markerloci sxn2151s01 und sle3305s02 lokalisiert. In einer bevorzugten Ausführungsform liegen diese Markerloci bei 33,42 bzw. 35,15 cM auf Chromosom 1 (basierend auf der genetischen Karte ZR INT 1202) und basierend auf der physikalischen Genomkarte (Physmapv2) hat diese Region eine physikalische Größe von 215,4 kbp und liegt zwischen den Positionen 3185718 bp und 3401120 bp. Für beide oben genannte Markerloci wurden KASP-Marker (KASP^{™}, SNP Genotyping-Chemie von LGC Limited) entwickelt, mit welchen der zu detektierende SNP bzw. die entsprechende Referenzsequenz identifiziert werden kann. Die sxn2151s01-Markersequenz dargestellt in SEQ ID Nr. 24 und die sle3305s02-Markersequenz dargestellt in SEQ ID Nr. 26 zeigen das Vorhandensein des *gst*-Locus an; die sxn2151 s01-Markersequenz dargestellt in SEQ ID Nr. 25 und die sle3305s02-Markersequenz dargestellt in SEQ ID Nr. 27 zeigen die Referenzsequenz an, wobei sich die Markersequenzen jeweils an Nukleotidposition 21 unterscheiden und ein "G" an dieser Stelle bei dem gst-Locus-tragenden Genotyp zu finden ist und ein "A" an dieser Stelle bei dem Referenz-Genotyp KWS2320 zu finden ist.

Gemäß der vorliegenden Offenbarung kann das Segment etwa 50 bis 5000 kbp groß sein, vorzugsweise 100 bis 1000 kbp, mehr bevorzugt 100 bis 500 kbp und besonders bevorzugt 200 bis 250 kbp, wobei das Segment weitere proteinkodierende Gene aufweist, vorzugsweise 21 Gene.

Gemäß der vorliegenden Offenbarung handelt es sich bei dem nicht-mutierten Gen um das funktionelle Gen *BvCYPgst* aus *Beta vulgaris,* vorzugsweise aus *Beta vulgaris* subsp. *vulgaris* oder um ein funktionelles homologes, analoges oder orthologes Gen einer anderen Nutzpflanze beziehungsweise Kulturpflanze.

Der Fachmann kann weitere CYP*gst* Proteine der einschlägigen Literatur sowie Datenbanken unter Verwendung geeigneter Suchprofile und Computerprogramme für das Screening nach homologen Sequenzen bzw. für Sequenzvergleiche entnehmen. Darüber hinaus kann der Fachmann mittels herkömmlichen molekularbiologischen Techniken weitere CYP*gst* Protein kodierende DNA-Sequenzen selber herausfinden und im Rahmen der vorliegenden Erfindung einsetzen. So können beispielsweise geeignete Hybridisierungssonden von der Sequenz des CYP*gst* Gens abgeleitet und für das Screening von genomischen- und/oder cDNA Banken des gewünschten Organismus eingesetzt werden. Hierbei kann der Fachmann auf geläufige Hybridisierungs-, Klonierungs- und Sequenzierungsmethoden zurückgreifen, die beispielsweise in Sambrook et al., Molecular Cloning: A Laboratory Manual 3rd Ed. Cold Spring Harbor Laboratory Press, Cold Spring Harbor, NY, 2001 aufgeführt sind. Auch kann der Fachmann anhand bekannter Sequenzen Oligonukleotidprimer zum Amplifizieren von CYP*gst* Sequenzen synthetisieren und einsetzen.

Offenbart wird hierin bei dem homologen, analogen oder orthologen Gen ein Gen aus *Zea mays,* das vorzugsweise eine der in SEQ ID Nr.: 9 oder 10 gezeigte Nukleotidsequenz umfasst bzw. die in SEQ ID Nr.: 11 gezeigte Aminosäuresequenz kodiert, aus *Solanum tuberosum,* das vorzugsweise eine der in SEQ ID Nr.: 12 oder 13 gezeigte Nukleotidsequenz umfasst bzw. die in SEQ ID Nr.: 14 gezeigte Aminosäuresequenz kodiert, aus *Triticum aestivum,* das vorzugsweise die in SEQ ID Nr.: 15 gezeigte Aminosäuresequenz kodiert, aus *Helianthus annuus,* das vorzugsweise die in SEQ ID Nr.: 16 gezeigte Aminosäuresequenz kodiert, aus *Hordeum vulgare,* das vorzugsweise die in SEQ ID Nr.: 17 gezeigte Aminosäuresequenz kodiert, aus *Brassica napus,* das vorzugsweise die in SEQ ID Nr.: 18 gezeigte Aminosäuresequenz kodiert, aus *Brassica oleracea,* das vorzugsweise die in SEQ ID Nr.: 19 gezeigte Aminosäuresequenz kodiert, aus *Brassica rapa,* das vorzugsweise die in SEQ ID Nr.: 20 gezeigte Aminosäuresequenz kodiert, aus *Glycine max,* das vorzugsweise die in SEQ ID Nr.: 21 gezeigte Aminosäuresequenz kodiert, aus *Gossypium,* das vorzugsweise die in SEQ ID Nr.: 22 gezeigte Aminosäuresequenz kodiert, und aus *Sorghum bicolor,* das vorzugsweise die in SEQ ID Nr.: 23 gezeigte Aminosäuresequenz kodiert. Die genannten Pflanzen lassen sich den Nutzpflanzen und vorzugsweise den Kulturpflanzen zuordnen.

Eine Ausführungsform der erfindungsgemäßen Pflanze ist eine vorstehend beschriebene Pflanze, wobei das nicht-mutierte Gen (Wildtypgen) eine Nukleotidsequenz aufweist, welche ausgewählt wird aus der Gruppe bestehend aus einer Nukleotidsequenz, die die in SEQ ID Nr. 1, 2, gezeigte Nukleotidsequenz aufweist.

In einer Ausführungsform weist das nicht-mutierte Gen (Wildtypgen) eine Nukleotidsequenz auf, die die in SEQ ID Nr. 3 gezeigte Aminosäuresequenz kodiert.

Die Nukleotidsequenz kann unter Verwendung herkömmlicher Verfahren, die im Stand der Technik bekannt sind, beispielsweise durch ortsgerichtete Mutagenese, PCR-vermittelte Mutagenese, Transposon-Mutagenese, Genome Editing etc. Substitutionen, Deletionen, Insertionen, Additionen und/oder jede andere Änderung entweder allein oder in Kombinationen in das Gen eingeführt werden, die die Nukleotidsequenz zwar ändern, jedoch die gleiche Funktion erfüllen, wie die Ausgangssequenz.

Daher umfasst die Erfindung auch eine vorstehend beschriebene Pflanze, wobei die Nukleotidsequenz auch ein funktionelles Fragment von den in SEQ ID Nr. 1, 2 gezeigten Nukleotidsequenzen aufweisen kann. Die Begriffe "Fragment" umfasst Gene mit einer Nukleotidsequenz ausreichend ähnlich zu der oben genannten Nukleotidsequenz. Der Begriff "ausreichend ähnlich" bedeutet eine erste Nukleotidsequenz oder Aminosäuresequenz, die eine ausreichende oder minimale Anzahl an identischen oder äquivalenten Nukleotiden bzw. Aminosäureresten relativ zu einem zweiten Nukleotid bzw. einer zweiten Aminosäuresequenz hat. Im Hinblick auf die Aminosäuresequenz weist diese auch nach Änderung durch ein oben genanntes Verfahren eine gemeinsame Strukturdomäne auf und/oder besitzt gemeinsame funktionelle Aktivität. Nukleotidsequenzen oder Aminosäuresequenzen, die zumindest etwa 45%, zumindest etwa 50%, mindestens etwa 55%, mindestens etwa 60%, mindestens etwa 65%, mindestens etwa 70% aufweisen, mindestens etwa 75%, mindestens etwa 80%, mindestens etwa 85%, mindestens etwa 90%, mindestens etwa 91%, mindestens etwa 92%, mindestens etwa 93%, mindestens etwa 94%, mindestens etwa 95%, mindestens etwa 96%, mindestens etwa 97%, mindestens etwa 98%, mindestens etwa 99%, oder mindestens etwa 100% identisch ist, werden hier als ausreichend ähnlich definiert. Vorzugsweise wird bei den funktionellen Fragmenten eine ausreichend Ähnlichkeit begründet, wenn die Nukleotidsequenz bzw. Aminosäuresequenz im Allgemeinen die gleiche Eigenschaft aufweist, wie die zuvor benannten Nukleotid- bzw. Aminosäuresequenzen der vorliegenden Erfindung.

Entsprechend weist das nicht-mutierte Gen (Wildtypgen), welches in der Pflanze umfasst wird, in einer Ausführungsform eine Nukleotidsequenz auf, die in der Lage ist an eine Nukleotidsequenz, die komplementär zu einer Nukleotidsequenz der SEQ ID Nr. 1, 2 bzw. zu der Nukleotidsequenz die die in SEQ ID Nr. 3 gezeigte Aminosäuresequenz kodiert, unter stringenten Bedingungen zu hybridisieren. Des Weiteren umfasst eine weitere Ausführungsform das nicht-mutierte Gen (Wildtypgen), das eine Nukleotidsequenz umfasst, welche eine Aminosäuresequenz kodiert, die gegenüber der in SEQ ID Nr. 3 gezeigten Aminosäuresequenz Abweichungen in Form von Aminosäure-Deletionen, Substitutionen, Additionen und/oder Insertionen in der Aminosäuresequenz aufweist, vorzugsweise nicht mehr als 20%, 15%, 10%, 5%, 4%, 3%, 2% oder nicht mehr als 1% über die gesamte Aminosäuresequenz.

In einer anderen bzw. zusätzlichen Ausführungsform kodiert die Nukleotidsequenz des nicht-mutierten Gens (Wildtypgen) ein Protein mit der gleichen enzymatischen Aktivität wie das Protein, das durch die DNA der vorangehenden Ausführungsformen kodiert wird.

In einer weiteren Ausführungsform weist das nicht-mutierte Gen (Wildtypgen), welches in der Pflanze umfasst wird, eine DNA auf, die mindestens 200 oder 400, bevorzugt mindestens 600 oder 800, besonders bevorzugt mindestens 1000 konsekutive Nukleotide aus dem Promotor der Nukleinsäuresequenz von SEQ ID Nr. 1 von Nukleotidpositionen 1 bis 1518, bevorzugt von Nukleotidpositionen 518 bis 1518, besonders bevorzugt von Nukleotidpositionen 1318 bis 1518 oder eine Sequenz, die an diesen Bereich hybridisiert, umfasst, wobei die Nukleotidsequenz in der Lage ist, ist Expression des Gens bzw. eines heterologen Nukleinsäuremoleküls, das operativ mit der DNA verknüpft ist, spezifisch in geschlossenen Blüten und/oder Früchten zu steuern.

Gemäß der vorliegenden Offenbarung kann es sich bei der Pflanze um eine Inzuchtpflanze oder eine Hybridpflanze handeln. Die Inzuchtpflanze kann als Elternpflanze zur Erzeugung von Hydriden genutzt. Der Vorteil der Verwendung einer für das Merkmal der rezessiven, kernkodierten männlichen Sterilität heterozygoten Inzuchtpflanze ist, dass sich diese in jeden Vermehrungsschritt in fertile und sterile Individuen aufspaltet. Das männlich sterile Individuum kann zur Erzeugung von Hybriden genutzt werden, womit eine manuelle Entfernung der Antheren entfällt und auch eine parallele Erhaltung einer sterilen *Maintainer-*Linie ist dadurch nicht mehr nötig.

Eine weitere Ausführungsform der vorliegenden Erfindung umfasst nicht nur die erfindungsgemäße Pflanze, die eine Mutation in dem CYP*gst* Gen aufweist, sondern auch ein DNA-Molekül, das eine wie zuvor definierte Nukleotidsequenz mit einer Mutation in Form einer Deletion, Addition, Insertion oder Substitution aufweist, wobei diese Mutation dazu führt, dass kein funktionsfähiges CYP*gst* Protein synthetisiert wird.

In einer bevorzugten Ausführungsform ist die Mutation in der kodierenden Nukleotidsequenz des CYP*gst* Gens, einem Splicing-Signal oder in einer regulatorischen Sequenz des CYP*gst* Gens, vorzugsweise im Promotor des CYP*gst* Gens lokalisiert. Insbesondere kann es sich bei der Mutation um eine Deletion zwischen den Nukleotidpositionen 1560 und 2095 der SEQ ID Nr.: 1 handeln. Die Deletion kann eine Länge von mindestens 20, 30 oder 50 konsekutiven Basenpaaren, bevorzugt von mindestens 100, 150, 200 oder 250 konsekutiven Basenpaaren und besonders bevorzugt von mindestens 300, 400 oder 500 konsekutiven Basenpaaren aufweisen. In einer weiteren bevorzugten Ausführungsform weist das Nukleinsäuremolekül eine Punktmutation in der Nukleotidsequenz der SEQ ID Nr. 1 gemäß der Tabelle 1 auf, vorzugsweise zwischen den Nukleotidpositionen 1560 und 2095 der SEQ ID Nr.: 1.

Wie zuvor beschrieben, können DNA-Hybridisierungssonden, die von der Sequenz des CYP*gst* Gens abgeleitet sind, für das Screening von genomischen- und/oder cDNA Banken anderer Organismen zum Identifizieren von homologen Genen eingesetzt werden. Um eine spezifische Hybridisierung zu erzielen, sollten solche Sonden spezifisch sein und mindestens eine Länge von 15 Nukleotiden, bevorzugt mindestens 20 Nukleotide aufweisen. Die Sonden können zum Amplifizieren der identifizierten homologen Gene durch den bekannten Prozess der Polymerase Kettenreaktion (PCR) eingesetzt werden. Des Weiteren können diese Sonden auch zur Detektion von Mutationen im CYP*gst* Gen eingesetzt werden. Eine ausführliche Anleitung zu der Hybridisierung von Nukleinsäuren kann man in Tijssen, Laboratory Techniques in Biochemistry and Molecular Biology - Hybridization with Nucleic Acid Probes, Teil 1, Kapitel 2, "Overview of principles of hybridization and the strategy of nucleic acid probe assays," Elsevier, New York (1993); und in Current Protocols in Molecular Biology, Kapitel 2, Ausubel, et al., eds, Greene Publishing and Wiley Interscience, New York (1995) finden.

Daher ist ein Nukleinsäuremolekül von mindestens 15,16, 17, 18, 19 oder 20, bevorzugt mindestens 21, 22, 23, 24 oder 25, besonders bevorzugt mindestens 30, 35, 40, 45 oder 50, und ganz besonders bevorzugt mindestens 100, 200, 300, 500 oder 1000 Nukleotiden Länge Gegenstand der vorliegenden Erfindung, wobei dieses Nukleinsäuremolekül spezifisch an eine zuvor beschriebene Nukleotidsequenz, die das nicht-mutierte CYP703 Wildtypgen umfasst oder an ein zuvor beschriebenes DNA-Molekül mit einer Mutation in Form einer Deletion, Addition, Insertion oder Substitution, die dazu führt, dass kein funktionsfähigen CYP703 Protein gebildet wird, hybridisiert.

Durch die oben beschriebene Feinkartierung konnte die Position des CYP*gst* Gens im Genom von *Beta vulgaris* subsp. *vulgaris* bestimmt werden. Dies wiederum stellte die Grundlage für die Entwicklung von genetischen Markern, mit deren Hilfe die Deletion im CYP*gst* Gen nachgewiesen werden konnte.

Daher betrifft die vorliegende Erfindung neben den oben beschriebenen Pflanzen auch Marker als Oligonukleotide, insbesondere Primer-Oligonukleotide. Diese umfassen ein Nukleinsäuremolekül von mindestens 15 Nukleotiden Länge, das spezifisch an eine wie zuvor definierte Nukleotidsequenz oder an ein zuvor definiertes DNA-Molekül mit einer Mutation in Form einer Deletion, Addition, Insertion oder Substitution, die dazu führt, dass kein funktionsfähigen CYP*gst* Protein gebildet wird, hybridisiert. Vorzugsweise weisen diese Oligonukleotide eine Länge von maximal 50 Nukleotiden auf. Weiter bevorzugt sind die Oligonukleotide kürzer und weisen eine Länge zwischen 15 und 25 Nukleotiden auf. Wie in Beispiel 2 der vorliegenden Erfindung gezeigt, weisen die Oligonukleotide bevorzugt eine der folgenden Nukleotidsequenzen auf: (i) SEQ ID Nr. 4, 6 oder ein Komplement davon, oder (ii) SEQ ID Nr. 5, 7 oder ein Komplement davon.

Hierin offenbart ist ferner ein CYPgst Protein kodierbar durch eine zuvor beschriebene Nukleotidsequenz und ein funktionelles und/oder immunologisch aktives Fragment davon sowie ein Antikörper, der spezifisch an das CYPgst Protein oder an dessen hier beschriebenen Fragment bindet. Die rekombinante Herstellung von Proteinen und Fragmenten ist dem Fachmann geläufig und beispielsweise in Sambrook et al., Molecular Cloning: A Laboratory Manual 3rd Ed. Cold Spring Harbor Laboratory Press, Cold Spring Harbor, NY, 2001 oder Wingfield, P. T. 2008. Production of Recombinant Proteins. Current Protocols in Protein Science. 52:5.0:5.0.1-5.0.4 beschrieben. Polyklonale oder monoklonaler Antikörper zu dem Protein können von dem Fachmann nach bekannten Verfahren hergestellt werden, wie sie beschrieben sind in E. Harlow et al., Herausgeber Antibodies: A Laboratory Manual (1988). Die Herstellung von monoklonalen Antikörpern sowie von Fab- und F(ab')₂-Fragmenten, die auch bei Proteindetektionsmethoden nützlich sind, kann durchgeführt werden durch verschiedene gebräuchliche Methoden wie sie beschrieben sind in Goding, Mononoclonal Antibodies: Principles and Practice, S. 98-118, New York: Academic Press (1983). Der Antikörper kann dann für das Screening von Expressions-cDNA-Bibliotheken genutzt werden um identischen, homologe oder heterologe Gene mittels immunologischen Screening zu identifizieren (Sambrook et al., Molecular Cloning: A Laboratory Manual, Cold Spring Harbor Laboratory Press, Cold Spring Harbor, NY, 1989 oder Ausubel et al., 1994, "Current Protocols in Molecular Biology", John Wiley & Sons).

Bei dem CYPgst-Protein handelt es sich um die in SEQ ID Nr. 3 aufgeführten Aminosäuresequenzen, oder um eine Aminosäuresequenz, die mit der in SEQ ID Nr. 3 aufgeführten Aminosäuresequenz zu mindestens 80%, 82%, 84%, 86% oder 88%, bevorzugt zu mindestens 90%, 91%, 92%, 93%, 94% oder 95%, besonders bevorzugt zu mindestens 96%, 97%, 98%, 99% oder 99,5%, vorzugsweise über die Volllänge identisch ist.

Offenbart wird weiterhin ein rekombinantes DNA-Molekül, das das nicht-mutierte CYP*gst* Gen (Wildtypgen) umfasst und die zuvor erwähnten Eigenschaften der Nukleotidsequenz aufweist. Bevorzugt weist das rekombinante DNA-Molekül einen Promotor und/oder andere Transkriptions- oder Translationskontrollelemente auf bzw. ist mit diesen assoziiert. Bei den verwendeten Promotoren wird es sich hauptsächlich um zellspezifische Promotoren handeln, die die Transkription der DNA nur in vorbestimmten Zellen ermöglichen. Neben den Promotoren gibt es eine Vielzahl weiterer Transkriptionskontrollelemente, wie beispielsweise die Enhancer, Operatoren, Repressoren und Transkriptionsterminationssignale, jedoch nicht auf diese beschränkt, welche mit der DNA funktionell verbunden sind, um eine gerichtete zellspezifische Transkription zu ermöglichen. Promotoren und andere Transkriptionsregulationselemente sind allgemein bekannt und dem Fachmann im Stand der Technik zugänglich; siehe beispielsweise WO 00/75359 auf Seite 23, Zeile 5 bis Seite 24, Zeile 17. Dieses rekombinante DNA-Molekül kann dafür verwendet werden, um in Pflanzen mit einem kernkodierten, rezessiven, männlich sterilen Phänotyp die Fertilität zu restaurieren.

Da, wie zuvor beschrieben, das CYP*gst* Gen in geschlossenen Blüten und Früchten exprimiert wird und nicht in Wurzeln und/oder Blättern, umfasst das rekombinante DNA-Molekül in einer bevorzugten Ausführungsform entweder einen Promotor, der eine zuvor beschriebene Nukleotidsequenz aufweist und mit einem heterologen Nukleinsäuremolekül operativ verknüpft ist oder eine wie zuvor definierte kodierende Nukleotidsequenz, die das Wildtyp-CYPgst Gen umfasst und die operativ mit einem heterologen Promoter verknüpft ist. Vorzugsweise ist dieser Promotor in der Lage, die Expression der Nukleotidsequenz spezifisch in geschlossenen Blüten und/oder Früchten zu steuern. Weiter bevorzugt umfasst das rekombinante DNA-Molekül den nativen Promoter des nicht-mutierten CYPgst Gens aus *Beta vulgaris* subsp. *vulgaris* (SEQ ID Nr. 1).

Dementsprechend umfasst die vorliegende Erfindung auch die Verwendung des hier beschriebenen DNA-Moleküls bzw. des hier beschriebenen Promotors zur spezifischen Expression von heterologen Nukleinsäuremolekülen in Blüten und/oder Früchten von Pflanzen. Dazu ist eine operative Verknüpfung von dem heterologen Nukleinsäuremolekül mit dem entsprechenden Promotor nötig und die Einbringung dieses rekombinanten DNA-Moleküls in die Ziel-Zelle, die vorzugsweise eine Pflanzenzelle ist. Verfahren zur heterologen Expression von rekombinanten DNA-Molekülen werden später genauer beschrieben.

Ein weiterer Gegenstand der vorliegenden Erfindung ist ein rekombinantes DNA-Molekül, das eine erfindungsgemäße Nukleotidsequenz aufweist, die eine shRNA (*small hairpin RNA*)*,* siRNA (*small interfering RNA*)*,* Antisense-RNA, Sense-RNA oder doppelsträngige RNA kodiert. Diese vermitteln durch Basenpaarung die Inhibierung der Translation der *CYPgst* mRNA oder den Abbau der CYPgst mRNA in der Zelle. Demnach führt das Einbringen und/oder die Expression des rekombinanten DNA-Moleküls in einer Pflanze zur Inhibierung der Expression des funktionellen (nicht-mutierten) CYPgst Gens.

Hierin offenbart werden Vektoren, die rekombinante DNA-Moleküle oder Nukleinsäuresequenzen bzw. Nukleinsäuremoleküle umfassen. Ein Vektor kann das nicht-mutierte CYPgst Gen (Wildtypgen) mit den zuvor erwähnten Eigenschaften der Nukleotidsequenz und vorzugsweise einen der zuvor beschriebenen Promotoren enthalten. Ein anderer Vektor kann ein rekombinantes DNA-Molekül enthalten, das den Promotor des nicht-mutierten CYP*gst* Wildtyp-Gens, der mit einem heterologen Nukleinsäuremolekül verknüpft ist, umfasst oder kann ein rekombinantes DNA-Molekül enthalten, das eine zuvor beschriebene Nukleotidsequenz umfasst, die operativ mit einem heterologen Promotor verknüpft ist, wobei in beiden Fällen der Promotor vorzugsweise in die Lage ist, die Expression der Nukleotidsequenz spezifisch in geschlossenen Blüten und/oder Früchten zu steuern. Des Weiteren kann ein Vektor ein rekombinantes DNA-Molekül enthalten, dass eine Nukleotidsequenz aufweist, die für eine shRNA, siRNA, Antisense-RNA, Sense-RNA oder doppelsträngige RNA kodiert und somit nach Expression in einer Pflanzenzelle zur Inhibierung der Expression des CYP*gst* Gens führt.

Weiterhin kann ein Vektor ein DNA-Molekül mit einer zuvor definierten Mutation enthalten oder kann das zuvor beschriebene Nukleinsäuremolekül enthalten, das spezifisch an die nicht-mutierte (Wildtyp) CYP*gst* Nukleotidsequenz oder an die mutierte CYP*gst* Nukleotidsequenz bindet.

Bei dem beschriebenen Vektor kann es sich um ein Plasmid, ein Cosmid, eine Phage oder einen Expressionsvektor, einen Transformationsvektor, Shuttle-Vektor oder Klonierungsvektor handeln, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist das erfindungsgemäße DNA-Molekül oder Nukleinsäuremolekül in einem Expressionsvektor mit einer oder mehreren regulatorischen Sequenzen, welche die Transkription und optional die Expression in einer prokaryotischen oder eukaryotischen Wirtszelle erlauben, operativ verknüpft; siehe beispielsweise Sambrook et al., Molecular Cloning: A Laboratory Manual 3rd Ed. Cold Spring Harbor Laboratory Press, Cold Spring Harbor, NY, 2001 und die internationale Anmeldung WO 00/75359 auf Seite 21, Zeile 20 bis Seite 22, Zeile 32. Vorzugsweise sind diese regulatorischen Sequenzen Promotoren oder Terminatoren insbesondere ein Transkriptions-Initiations-Startpunkt, eine Ribosomen-Bindestelle, ein RNA-prozessierendes Signal, eine Transkriptions-Terminations Stelle und/oder ein Polyadenylierungssignal. Die Vektoren enthalten zusätzlich für gewöhnlich Indikator-/Reportergene oder Resistenzgene zum Nachweisen der Übertragung des gewünschten Vektors bzw. DNA-Moleküls/Nukleinsäuremoleküls und zum Selektieren der Individuen die diese enthalten, da ein direkter Nachweis über die Expression des Gens meinst eher schwierig ist. Beispiele für Indikator-/Reportergene sind beispielsweise das Luciferase-Gen und das Gen kodierend für das Grün-fluoreszierende-Protein (GFP). Diese erlauben ferner auch Untersuchungen zur Aktivität und/oder Regulation eines Promotors des Gens. Beispiele für Resistenzgene, im speziellen für Pflanzentransformationen sind das Neomycin-Phosphotransferase-Gen, das Hygromycin-Phosphotransferase Gen oder das Gen kodierend für die Phosphinothricin-Acetyltransferase. Dies schließt weitere dem Fachmann bekannte Indikator-/Reportergene oder Resistenzgene jedoch nicht aus. In einer bevorzugten Ausführungsform ist der Vektor ein Pflanzenvektor.

Offenbart wird auch ein zuvor beschriebener Vektor, wobei das DNA-Molekül als Transgen in der Lage ist ein funktionelles CYP*gst* Gen zu exprimieren und vorzugsweise genetisch gekoppelt ist mit einem weiteren Transgen, welches die Weitergabe des DNA-Moleküls über den Pollen verhindert. Durch Einbringung dieses Vektors in eine Mutante, die durch eine Mutation in dem CYP*gst* Gen männlich steril ist, kann die Fertilität wieder restauriert werden. Dadurch, dass die Weitergabe des transgenen funktionellen CYPgst Gens über die Pollen verhindert wird, entsteht bei der Selbstbefruchtung der transgenen Linie nur hemizygotes Saatgut.

Vorzugsweise weist der Vektor bzw. das Transgen des Weiteren eine Expressionskassette auf, die zu einer Markierung der Samen führt, vorzugsweise durch Fluoreszenz-Markierung. Durch diesen Ansatz lassen sich transgene von nicht-transgenen Samen einfach unterscheiden. Dieses System für die Nutzung von kerncodierter, männlicher Sterilität wurde von der Firma Pioneer entwickelt. Das System mit dem Namen SEED PRODUCTION TECHNOLOGY (SPT) (US 2006288440 A1) wurde für Mais entwickelt und basiert darauf, dass eine sterile Mutante, die durch eine Mutation in einem bekannten, kerncodierten Gen durch Einfügen eines Transgens restauriert werden kann. Das Transgen enthält dabei das nicht mutierte Allel des Sterilitätsgens, so dass das Transgen als wildtyp-Allel fungiert. Das Fertilität restaurierende Transgen ist genetisch gekoppelt mit einem weiteren Transgen, welches die Weitergabe des Transgens über den Pollen verhindert (Pollenkiller). Dadurch entsteht bei Selbstbefruchtungen der transgenen Linie nur hemizygotes Saatgut, was für die Effizienz des Systems sehr wichtig ist.

Das Transgen enthält des Weiteren eine Expressionskassette, die zur Rotfluoreszenz der Samen führt. Somit lassen sich transgene von nicht transgenen Samen einfach unterscheiden. Da die transgenen Samen fertil sind, findet dadurch auch automatisch eine Separierung in fertile und sterile Pflanzen statt. Durch Aussaat der nicht transgenen Samen erhält man somit die für die Hybridproduktion notwendigen Mutterpflanzen, die transgenen Samen können zum einen als Vaterlinie für die weitere Vermehrung der Mutterlinie verwendet werden (*Maintainer*-Linie) und auch durch einfache Selbstung vermehrt werden (Abbildung 5). Das SPT System kann theoretisch in allen Pflanzenarten angewandt werden. Voraussetzung ist jedoch das Vorhandensein einer genisch männlich sterilen Linie sowie die Kenntnis über das Gen, welches für den genisch männlichen Sterilitätsphänotyp verantwortlich ist. Somit könnte das SPT-System theoretisch auch für die Entwicklung eines Hybridsystems für jede Kulturart wie Zuckerrübe oder Kartoffel verwendet werden.

Offenbart werden auch Wirtszellen, die die beschriebenen Vektoren, rekombinante DNA-Moleküle und/oder Nukleinsäuremoleküle enthalten. Eine Wirtszelle kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium oder eine Pflanzenzelle. Die vorliegende Erfindung betrifft eine transgene Pflanzenzelle, die das erfindungsgemäße Nukleinsäuremolekül als Transgen oder den Vektor der vorliegenden Erfindung umfasst. Eine solche transgene Pflanzenzelle ist beispielsweise eine Pflanzenzelle, welche mit dem erfindungsgemäßen Nukleinsäuremolekül oder mit dem Vektor der vorliegenden Erfindung, vorzugsweise stabil, transformiert ist. In einer bevorzugten Ausgestaltung der transgenen Pflanzenzelle ist das Nukleinsäuremolekül mit einer oder mehreren regulatorischen Sequenzen, welche die Transkription und optional die Expression in der Pflanzenzelle erlauben, operativ verknüpft. Das Gesamtkonstrukt aus dem erfindungsgemäßen Nukleinsäuremolekül und der/den regulatorischen Sequenzen stellt dann das Transgen dar. Solche regulatorische Sequenzen sind beispielsweise ein Promotor oder ein Terminator. Dem Fachmann sind zahlreiche in Pflanzen anwendbare, funktionelle Promotoren und Terminatoren bekannt.

In einem zusätzlichen Aspekt der Erfindung wird die Identifizierung des *CYPgst* Gens, verantwortlich für die Merkmalsausprägung der rezessiven, kernkodierten männlichen Sterilität, dazu genutzt, transgene Pflanzen mit dieser Merkmalsausprägung herzustellen und dazu genutzt um die Fertilität zu restaurieren.

Hierin offenbart wird ein Kit, das die erforderlichen und hier zuvor beschriebenen rekombinanten DNA-Moleküle bzw. Nukleinsäuremoleküle und Vektoren umfasst um Pflanzen mit kernkodierter, rezessiver männlicher Sterilität herzustellen und auch um in Pflanzen die diesen Phänotyp aufweisen die Fertilität zu restaurieren. Auch enthält dieses Kit rekombinante DNA-Moleküle, die entweder den Promotor mit einer zuvor definierten Nukleotidsequenz oder einen heterologen Promotor umfassen, wobei Ersterer mit einem heterologen Nukleinsäuremolekül und Letzterer mit einer zuvor definierten Nukleotidsequenz, die für das nicht-mutierte (Wildtyp) CYP*gst* Gen kodiert, verknüpft ist. Des Weiteren kann das Kit einen Vektor enthalten, wobei das DNA-Molekül als Transgen in der Lage ist ein funktionelles CYPgst Gen zu exprimieren und vorzugsweise genetisch gekoppelt ist mit einem weiteren Transgen, welches die Weitergabe des DNA-Moleküls über den Pollen verhindert und eine Expressionskassette aufweist, die zu einer Markierung der Samen führt. Zur Identifizierung der Mutation in dem CYPgst Gen kann das Kit ein zuvor definiertes Nukleinsäuremolekül enthalten, das an eine vorstehend beschriebene Nukleotidsequenz umfassend das nicht-mutierte (Wildtyp) CYPgst Gen oder an das entsprechende Gen mit einer zuvor definierten Mutation, hybridisiert oder es kann die zuvor definierten Oligonukleotide enthalten. Das Kit kann weiterhin das zuvor beschriebene CYP*gst* Protein oder Fragment davon sowie den beschriebenen Antikörper enthalten. Vorzugsweise sind in dem Kit auch Reagenzien für nukleinsäurebasierte oder immunologischen Nachweiseverfahren enthalten.

Eine transgene Pflanze ist beispielsweise eine Pflanze, die Pflanzenzellen enthält, welche mit dem erfindungsgemäßen DNA-Molekül/Nukleinsäuremolekül oder mit dem Vektor der vorliegenden Erfindung transformiert ist. In einer bevorzugten Ausführungsform der transgenen Pflanze ist das DNA-Molekül/Nukleinsäuremolekül mit einer oder mehreren regulatorischen Sequenzen, welche die Transkription und optional die Expression in der Pflanze erlauben, operativ verknüpft. Das Gesamtkonstrukt aus dem erfindungsgemäßen Nukleinsäuremolekül und der/den regulatorischen Sequenzen stellt dann das Transgen dar. Unter dem Begriff "Transgen" wird somit die ein rekombinantes Polypeptid kodierende Nukleinsäuresequenz verstanden.

Zur Herstellung einer transgenen Pflanze können die zuvor beschriebenen Oligonukleotide, Nukleinsäuren, DNA-Moleküle und Vektoren, auch dienlich sein. Daher betrifft die vorliegende Erfindung auch die Verwendung dieser in der Herstellung einer transgenen Pflanze die einen rezessiven, kernkodierten, im homozygoten Zustand männlich sterilen Phänotyp zeigt, dadurch gekennzeichnet, dass die Expression des CYP*gst* Gens inhibiert wird, in der Herstellung einer erfindungsgemäßen Pflanze mit restaurierter Fertilität oder in der Herstellung einer transgenen Pflanzenzelle. Zudem finden die zuvor beschriebenen Oligonukleotide, Nukleinsäuren, DNA-Moleküle und Vektoren auch Einsatz in den entsprechenden Verfahren zur Herstellung dieser transgenen Pflanzen oder Pflanzenzellen. Die transgene Pflanze ist vorzugsweise eine Nutzpflanze und weiter bevorzugt eine Kulturpflanze.

Es gibt verschiedenen Verfahren im Stand der Technik, mit denen sich transgene Pflanzen, in denen entweder die Transkription/Translation eines Proteins unterbunden wird oder das Restaurationsmerkmal eingebracht wird, herstellen und identifizieren bzw. selektieren lassen. Verfahren zur Herstellung von transgenen Kulturpflanzen und deren Identifizierung durch molekularbiologische Methoden sind dem Fachmann bekannt; siehe beispielsweise für transgene glyphosatresistente Zuckerrüben die internationale Anmeldung WO 99/023232 und WO2004/074492 bzw. für Transformation von Pflanzen im allgemeinen WO2000/018939 und WO2013/138309.

Eine Ausführungsform der vorliegenden Erfindung ist demnach ein Verfahren zur Herstellung einer Pflanze die einen rezessiven, kernkodierten, im homozygoten Zustand männlich sterilen Phänotyp zeigt, dadurch gekennzeichnet, dass die Expression des CYPgst Gens inhibiert wird,. Zur Erstellung dieser erfindungsgemäßen transgenen Pflanze kann ein rekombinantes DNA-Molekül, das ein Polynukleotid exprimiert, beispielsweise mit Hilfe eines Vektor in die Pflanzenzelle mittels Transformation eingebracht werden, sodass die Expression des Polynukleotids zur Inhibierung des CYPgst Proteins führt.

Beispielsweise kann die zuvor beschriebene Mutation, die zur Inhibierung der CYP*gst* Expression führt, durch genetische Rekombination während eines Kreuzungsprozesses zwischen zwei Pflanzen erreicht werden, wobei eine Pflanze das mutierte CYPgst Allel trägt. Neben der Verwendung von konventionellen Züchtungstechniken zur Erzeugung einer genetischen Rekombination stellt die moderne Biotechnologie dem Fachmann diverse weitere Werkzeuge zur Verfügung, welche ein präzises Genom-Engineering ermöglichen. Beispielweise könnte das T-DNA-Tagging dafür genutzt werden um das *CYPgst* Gen durch Insertionsmutagenese zu zerstören. Weiterhin könnte das CYP*gst* Gen durch Genmutation vollständig oder teilweise mittels TALE-Nukleasen (TALENs) oder Zinkfinger-Nukleasen (ZFNs) sowie CRISPR/Cas Systeme, die u.a. beispielhaft in WO 2014/144155 Al (Engineering plant genomes using CRISPR/Cas systems) und in Osakabe & Osakabe, Plant Cell Physiol, 56 (2015), 389-400 beschrieben sind, deletiert werden, sodass eine Expression des *CYPgst* Gens ausgeschlossen ist. Dies könnte auch durch Verwendung der als TILLING (*Targeted Induced Local Lesions in Genomes*) bezeichneten Methode erreicht werden, wobei, wie es beispielweise in der deutschen Patentanmeldung DE 10 2013 101 617 beschrieben ist, Punktmutationen in dem Wildtypgen hervorgerufen und anschließend Pflanzen selektiert werden, die eine geeignete, d.h. resistenzvermittelnde Mutation aufweisen, wie z.B. eine gegen Gelbmosaikvirose resistente Gerste; siehe die DE 10 2013 101 617 auf Seiten 4, 8 und 12 in Paragraphen [0014], [0026] und [0038]. Die TILLING Methode ist auch ausführlich in der Publikation von Henikoff *et al.* beschrieben (Henikoff et al., Plant Physiol. 135, 2004, 630-636. Punktmutationen in dem CYPgst Gen von *Beta vulgaris* subsp. *vulgaris,* die zu Stoppcodons oder Splicing-Fehlern führen könnten, sind in Tabelle 1 aufgeführt.

Eine Inhibierung der Expression ist durch RNAi Ansatz oder Co-Suppression auch möglich. Dieser umfasst das Einbringen des zuvor definierten rekombinanten DNA-Moleküls bzw. Nukleinsäuremoleküls oder des entsprechenden Vektors in die Pflanze, wobei die Expression der kodierten shRNA-, Antisense-RNA- oder Sense-RNA-Moleküle zur Inhibierung der Expression des CYP*gst* Gens führen. Solche RNAi und/oder Co-Suppression basierten Verfahren sind gängige Methoden zur Inhibierung der Genexpression und sind dem Fachmann bekannt. Auch kann ein Sense Ansatz, umfassend ein zielspezifisches nicht-polyadenyliertes RNA-Molekül zur Inhibierung der Expression des CYP*gst* Gens führen. Diese Methode ist zum Beispiel in der internationalen Anmeldung WO2001/012824 beschrieben.

Eine weitere Ausführungsform der Erfindung ist ein Verfahren zur Restauration der Fertilität einer erfindungsgemäßen Pflanze, das das Einbringen eines funktionellen CYP*gst* Gens in die Pflanze umfasst. Dabei kann das CYP*gst* Gen durch gentechnische Methoden eingebracht werden; mittels einer erfindungsgemäßen rekombinanten DNA, die vorzugsweise Transkriptionskontrollelemente, vorzugsweise einen Promoter zur spezifischen Expression des Gens in geschlossenen Blüten und/oder Früchten enthält, oder mittels der erfindungsgemäßen Vektoren. Das CYP*gst* Gen kann aber auch durch Kreuzung mit einer Pflanze, die das CYP*gst* Wildtypgen oder ein funktionelles CYP*gst* Gen, vorzugsweise im homozygoten Zustand trägt, eingebracht werden. Optional kann nach der Kreuzung eine Selektion auf Anwesenheit des CYP*gst* Wildtypgens oder des funktionellen CYP*gst* Gens in der Nachkommengeneration erfolgen.

Gegenstand der vorliegenden Erfindung ist eine Pflanze, die eine zuvor definierte Pflanzenzelle enthält und/oder die durch die zuvor beschriebenen Verfahren erhalten wurde. Das heißt, eine Pflanze mit einem kernkodierten, rezessiven männlich sterilen Phänotyp sowie eine Pflanze mit restaurierter Fertilität wurde entweder durch genetische Rekombination unter Verwendung von konventionellen Züchtungsmethoden hergestellt oder sie ist eine transgene Pflanze in der durch die verschiedenen zuvor genannten Verfahren die Expression des CYP*gst* Gens inhibiert wurde, was zu einem kernkodierten, rezessiven, männlich sterilen Phänotyp führt oder in der die Fertilität durch Einbringen von rekombinanten DNA-Molekülen restauriert wurde.

Neben den Pflanzen die einen kernkodierten, rezessiven, männlich sterilen Phänotyp durch Mutation im CYP*gst* Gen aufweisen oder den Pflanzen in denen dieser Phänotyp durch Genom-Engineering unter Verwendung von moderner Biotechnologie herbeigeführt wurde und den Pflanzen in denen die Fertilität durch die entsprechenden Verfahren restauriert wurde, betrifft die Erfindung auch Organe, Pflanzenteile, Gewebe, Zellen sowie Samen oder Nachkommen dieser Pflanzen. In einer Ausführungsform weisen die Samen oder Nachkommen eine oder mehrere der zuvor definierten Mutationen auf, die zur Inhibierung der Expression des *CYPgst* Gens führen und/oder die Samen oder Nachkommen weisen ein zuvor beschriebenes rekombinantes DNA-Molekül bzw. Nukleinsäuremolekül oder Vektor auf.

In dieser Erfindung ist ein Verfahren zur Identifizierung einer erfindungsgemäßen Pflanze ebenfalls eine weitere Ausführungsform. Mit diesem Verfahren kann sowohl die Pflanze, die einen kernkodierten, rezessiven, männlich sterilen Phänotyp durch Mutation im CYP*gst* Gen aufweisen identifiziert werden oder eine Pflanze, in der dieser Phänotyp durch Genom-Engineering unter Verwendung von moderner Biotechnologie herbeigeführt wurde. Zudem kann eine Pflanze mit diesem Verfahren identifiziert werden, in der die Fertilität durch die entsprechenden Verfahren restauriert wurde. Zur Identifizierung dieser erfindungsgemäßen Pflanze kann ein zuvor definiertes Nukleinsäuremolekül als Hybridisierungssonde verwendet werden, das mindestens eine Länge von 15 Nukleotiden hat und spezifisch an eine zuvor definierte Nukleotidsequenz umfassend das nicht-mutierte (Wildtyp) CYP*gst* Gen sowie das CYP*gst* Gen mit einer zuvor definierten Mutation, die zur Inhibierung der Expression des Gens führt, binden. Offenbart wird hierin, dass für die Identifizierung das zuvor definierte Oligonukleotid, das zuvor definierte CYP*gst* Protein bzw. ein Fragment davon und der Antikörper sowie Bestandteile des zuvor beschriebenen Kits verwendet werden kann.

Die vorliegende Erfindung betrifft auch die Verwendung von erfindungsgemäßen rekombinanten DNA-Molekülen bzw. Nukleinsäuremolekülen in der Herstellung einer rezessiven, kernkodierten, männlich sterilen Pflanze, in der Herstellung einer Pflanze mit restaurierter Fertilität.

Ein Verfahren zur Herstellung reversibler männlicher Sterilität in einer Pflanze, in dem das CYP*gst* Gen genutzt werden kann, ist beispielsweise in der internationalen Anmeldung WO96/017945 beschrieben, umfassend:
(a) Einbringen eines ersten rekombinanten DNA-Moleküls in das Genom einer Pollenproduzierenden Pflanze, die genetisch transformiert werden kann, wobei das erste rekombinante DNA-Molekül umfasst:
   (i) eine Nukleotidsequenz, die ein Genprodukt kodiert, welches, nach Expression in einer Pflanze, die Pollenbildung oder deren Funktion hemmt, hier erfindungsgemäß das CYP*gst* Gen bzw. Genprodukt beispielweise durch Expression einer RNAi-Sequenz;
   (ii) einen Operator, der die Expression der Nukleotidsequenz kontrolliert; und
   (iii) einen Promotor, der spezifisch für Zellen ist, die kritisch für die Pollenbildung oder deren Funktion sind, wobei der Promotor funktionell mit der Nukleotidsequenz, die ein Genprodukt kodiert, verknüpft ist;
(b) optional Züchten der Pflanze, die in Schritt (a) erhalten wurde, unter Bedingungen, die es erlauben, dass männliche Sterilität als Ergebnis der Expression der Nukleotidsequenzen erreicht wird;
(c) Kreuzen der männlich sterilen Pflanze aus (a) oder (b) mit Pollen von einer männlichen fertilen Linie, um eine Hybridpflanze zu erzeugen, die männlich fertil ist, wobei die Pollen ein zweites rekombinantes DNA-Molekül in ihr Genom integriert haben, wobei das zweite rekombinante DNA-Molekül umfasst: eine Nukleotidsequenz, die ein DNAbindendes Protein kodiert und die Repression der Transkription verursacht, und einen Promotor, der die Expression der Nukleotidsequenz kontrolliert, wobei das DNAbindende Protein in der Lage ist, den Operator der rekombinanten DNA der männlich sterilen Pflanze zu binden und die Repression der Transkription zu verursachen.

Ein weiteres System zur Herstellung von pollensterilen Pflanzen, in denen Fremd-DNA ins Kerngenom eingeführt wird und das erfindungsgemäß genutzt werden kann ist in der europäischen Patentanmeldung EP 0 344 029 beschrieben.

Offenbart wird hierin ferner die Verwendung der erfindungsgemäßen Pflanzen zur Züchtung oder zur Herstellung einer Nachkommenpflanze, wobei der kernkodierte männlich sterile Phänotyp zur rekurrenten Selektion verwendet wird. Offenbart werden auch Samen oder Nachkommen, oder Organ, Pflanzenteil, Gewebe oder Zellen davon in der Herstellung von üblicherweise aus nachwachsenden Rohstoffen gefertigten Produkten wie Nahrungsmittel und Futtermittel, vorzugsweise Zucker oder Sirup (Melasse), wobei die Melasse auch für industrielle Anwendungen genutzt wird, beispielsweise in der Alkoholgewinnung oder als Nährmedium für die Herstellung von biotechnologischen Produkten, in der Herstellung von Werkstoffen oder Stoffen für die chemischen Industrie, z.B. Feinchemikalien, Arzneimitteln bzw. Vorstufen davon, Diagnostika, Kosmetika, Bioethanol oder Biogas. Ein Beispiel für die Verwendung von Zuckerrübe als biogener Rohstoff in Biogasanlagen ist in der Anmeldung DE 10 2012 022 178 A1 beschrieben, siehe z.B. Absatz 10.

Offenbart wird hierin auch durch die Pflanzen, Organe, Pflanzenteile, Gewebe, Zellen, Samen und Nachkommen erhältliche Produkte wie Nahrungsmittel, Futtermittel und Werkstoff enthaltend eine erfindungsgemäße Pflanze, Samen, Nachkommen, Organe, Pflanzenteile, Gewebe oder Zellen bzw. Bestandteile davon.

Sofern nicht anders angegeben, wurden in den folgenden Beispielen molekularbiologischen Standardmethoden verwendet, siehe beispielsweise (Sambrook et al., Molecular Cloning: A Laboratory Manual 3rd Ed. Cold Spring Harbor Laboratory Press, Cold Spring Harbor, NY, 2001), Fritsch *et al.,* Cold Spring Harbor Laboratory Press: 1989; Mayer et al., Immunochemical Methods In Cell And Molecular Biology, eds., Academic Press, London, 1987) und Weir et al., Handbook Of Experimental Immunology, Volumes I-IV, Blackwell, eds., 1986).

### BEISPIELE

### 1. Identifizierung eines Locus, der kernkodierte männliche Sterilität verursacht

Als Ausgangspflanze zur Identifizierung eines Locus, der kernkodierte männliche Sterilität in Zuckerrübe verursacht, wurde ein Donor mit interner Bezeichnung C311 [2043_K5] verwendet, der einen rezessiven, kernkodierten, männlich sterilen Phänotyp (Arbeitsname *gst*) zeigt. Die Anwesenheit und der Zygotiegrad des von diesem Donor stammenden und der Merkmalsausprägung zu Grunde liegenden gst-Locus ließen sich im Zuchtmaterial jedoch nicht vorab (also vor der Blüte) prüfen. Vielmehr mussten eine große Anzahl von putativ sterilen Pflanzen im Feld, bzw. Selbstungs-Block (S-Block), zur Blüte gebracht werden. Blühende Pflanzen wurden dann manuell für die Merkmale Fertilität bzw. Sterilität bonitiert (Abb. 1). Im Anschluss konnten fertile Individuen entfernt und Saatgut steriler Individuen geerntet werden.

Zur genetischen und physikalischen Eingrenzung des gst-Locus, der die kernkodierte männliche Sterilität verursacht, wurde eine für das Merkmal spaltende Zuckerrüben-Kartierungspopulation erstellt. Aus ersten Vorinformationen einer genomweiten Kartierung war bereits die Zielregion auf Chromosom 1 bekannt. Zur weiteren Feinkartierung wurden männlich sterile Individuen des gst-Donors C311 [2043_K5] mit einer annuellen Linie gekreuzt und die resultierenden F1 Individuen wurden durch Selbstbefruchtung vermehrt. Für die Kartierung wurden schließlich Nachkommen der S1 Generation phänotypisiert und durch KASP-DNA Marker (KASP^{™}, SNP Genotyping-Chemie von LGC Limited) charakterisiert. Es wurden 2 KASP-DNA Marker, sxn2151s01 und s1e3305s02, entwickelt, wodurch der *gst-*Locus-tragenden Genotyp von dem Referenz-Genotyp KWS2320 durch Identifizierung eines SNPs unterschieden werden kann. Die sxn2151s01-Markersequenz dargestellt in SEQ ID Nr. 24 und die sle3305s02-Markersequenz dargestellt in SEQ ID Nr. 26 zeigen das Vorhandensein des gst-Locus an; die sxn2151s01-Markersequenz dargestellt in SEQ ID Nr. 25 und die sle3305s02-Markersequenz dargestellt in SEQ ID Nr. 27 zeigen die Referenzsequenz an, wobei sich die Markersequenzen jeweils an Nukleotidposition 21 unterscheiden und ein "G" an dieser Stelle bei dem gst-Locus-tragenden Genotyp zu finden ist und ein "A" an dieser Stelle bei dem Referenz-Genotyp KWS2320 zu finden ist. Als Ergebnis dieser Feinkartierung wurde die Region auf Chromosom 1 des Zuckerrübengenoms stark eingegrenzt, die von den KASP-DNA Markern sxn2151s01 bei 33,42 cM und s1e3305s02 bei 35,15 cM (basierend auf der genetischen Karte ZR INT 1202) flankiert wird und den *gst*-Locus trägt. Basierend auf der physikalischen Genomkarte (Physmapv2) hat diese Region eine physikalische Größe von 215,4 kbp und liegt zwischen den Positionen 3185718 bp und 3401120 bp. Basierend auf der identifizierten Position und der öffentlich erhältlichen Genomannotation RefBeet 1.2 (http://bvseq.molgen.mpg.de/) wurden 21 proteinkodierende Gene identifiziert, die in diesem Genomabschnitt lokalisiert sind.

Zu allen 21 Genmodellen wurden mittels bioinformatorischer Ansätze Homologe Gene in Modellpflanzen (z.B. *Arabidopsis thaliana* und *Oryza sativa*) gesucht. Basierend auf den identifizierten Homologen in Modellpflanzen wurde eine umfassende Analyse und Auswertung vorgenommen. Aufgrund dieser umfassenden Analyse von Sequenz und den vorhergesagten Strukturen wurde ein der im gst-Lokus kodierten Gene als Mitglied der Familie der Cytochrom P450-Monooxygenasen (CYPs) identifiziert. Trotz beträchtlicher Sequenzdiversität der Cytochrom P450-Monooxygenasen verfügen alle CYPs über gemeinsame strukturelle Merkmale, die besonders im Bereich des aktiven Zentrums hoch konserviert sind (siehe bspw. Fischer et al., Bioinformatics 23 (2007), 2015-2017), und die auch für das putative *gst*-Gen gefunden wurden. Daher wurde dieses Gen CYP*gst* genannt.

Bei näherer Charakterisierung dieses Gens wurde ein *Arabidopsis thaliana* CYP Gen identifiziert, d.h. *CYP703A2,* das eine hohe Sequenzidentität zu diesem Gen aus dem *gst*-Lokus aufwies. Eine *Arabidopsis thaliana* Mutante, in der dieses Gen durch Insertion einer T-DNA inaktiviert wurde, zeigt einen partiellen bzw. semi-männlich sterilen Phänotyp (Morant et al. Plant Cell 19 (2007), 1473-1487). Mechanistisch erklärt wird dies durch eine Funktion des CYP703A2 in der Synthese des Sporopollenins, dem Hauptbestandteil der Exin-Schicht vitaler Pollen. Eine fehlende Exin-Schicht unterbricht die Reifung der Pollen bzw. macht sehr empfindlich gegen Umwelteinflüsse. Es gibt jedoch zwei wesentlich Unterschiede im Phänotyp des Zuckerrüben *gst*-Phänotyps zum Phänotyp der beschriebenen *Arabidopsis* Mutante:
(i) im Gegensatz zu *Arabidopsis* erzeugt der *knockout* des Gens in Zuckerrüben eine vollständige männliche Sterilität und
(ii) während in *Arabidopsis* Mutanten Pollen grundsätzlich angelegt werden die allerdings steril sind, kommt es nach gegenwärtigem Stand der Analysen in *gst*-Zuckerrüben nicht zur Ausbildung von Pollen.

Daher ist nicht ausschließen, dass es sich um verschiedene Mitglieder der CYP Familie handelt und/oder die Funktionen beider Proteine in *Arabidopsis* und Zuckerrübe verschieden sind. Hinzu kommt, dass *Arabidopsis* ein Wildkraut aus der Familie der Kreuzblütler mit einem kompakten, kleinen Genom ist während die Zuckerrübe eine Kulturpflanze ist, d.h. eine vom Menschen angebaute, gepflegte und gezüchtete Pflanze, die als Nutzpflanze Verwendung findet. Daher lassen sich Experimente an *Arabidopsis* als Studienobjekt und deren Ergebnisse nicht ohne weiteres auf Kulturpflanzen übertragen. Zudem gibt es auch wichtige, landwirtschaftlich relevante Prozesse in Nutzpflanzen, die in *Arabidopsis* gar nicht vorkommen. Hierzu gehören beispielsweise die Ausbildung von Rübenkörpern, Knollen und Korn, die als Speicherorgan und als vegetatives Vermehrungsorgan dienen, Wechselwirkungen mit symbiontischen Mykorrhizapilzen oder Pathogenen, die nicht mit *Arabidopsis* in Verbindung stehen.

### 2. Charakterisierung des CYPgst Gens

Nach der Identifizierung des potenziellen Gens, das den *gst*-Phänotyp verursacht, erfolgte die vergleichende Sequenzierung eines etwa 5 kbp umfassenden Fragments genomischer DNA. Sequenziert wurden der fertile Zuckerrüben-Referenzgenotyp KWS2320, der sterile gst-Donor C311 und jeweils drei nach Phänotypen und Markerdaten als steril und drei als homozygot fertil klassifizierte Individuen der o.g. Feinkartierungspopulation. Die sequenzierte Genomregion umfasste das in Abbildung 2 dargestellte Genmodell von *BvCYP703A2* (*GST,* g6845.t1) und zusätzlich etwa 1,5 kbp der putativen Promotorregion. Die vergleichende Sequenzierung offenbarte neben einer Reihe von *Small Nucleotide Polymorphisms* (SNPs) zwischen sterilen und fertilen Individuen eine 533 bp Deletion in sterilen Genotypen, die die 5' UTR und das erste Exon des Genmodells umfasst (Abbildung 2 und Abbildung 3).

Die Analyse aller identifizierten Polymorphismen ergab, dass einzig die Deletion Auswirkungen auf das kodierte Protein hat, während alle anderen Mutationen entweder in untranslatierten Regionen liegen, oder synonyme Codons erzeugen. Die angesprochene Deletion hingegen verhindert eine korrekte Transkription der mRNA und die Translation eines funktionsfähigen Proteins ist damit ausgeschlossen. Im Anschluss durchgeführte Transkriptionsanalysen bestätigen diesen Befund (Abbildung 5). In fertilen Genotypen wird *BvCYP703A2* (*GST,* g6845.t1) sehr spezifisch in geschlossenen Blüten und Früchten exprimiert. In Wurzeln und Blättern ist keine Expression nachweisbar. In sterilen Genotypen hingegen ist keine Expression des *GST-*Gens in geschlossenen Blüten nachweisbar, sodass auf einen vollständigen knockout des Gens in sterilen Genotypen geschlossen werden kann.

Schließlich wurden DNA Marker entwickelt, die zwischen sterilen und fertilen Genotypen diskriminieren können. Dazu wurden KASP-Marker entwickelt, die dominant das fertile Allel (Insertion) anzeigen (sle5983d14, s1e5983d17).

| **maName** | **Primer_forward** | **Primer_reverse** |
|---|---|---|
| | **SEQ ID NO: 4:** | **SEQ ID NO: 5:** |
| s1e5983d14 | ACCAAAATTTTATACCAATGGCTCAAG | GGCCGGGAGGGAGTTTGTATGTT |
| s1e5983d17 | **SEQ ID NO: 6:** | **SEQ ID NO: 7:** |
| | AGAAATCATACGTGAGATCTTAGTTCG | GGTATGTGGACGAGACGCAAATACAT |

Durch diese kann indirekt auf die homozygot vorliegende Deletion geschlossen werden, wenn beide dominanten Marker (sle5983d14, s1e5983d17) ein Nullallel anzeigen und ein dritter, ubiquitärer Marker die hinreichende Qualität der DNA-Extraktion bestätigt. Potentielle Punktmutationen im *Bv*CYP*gst* Gen, die zu einem vorzeitigen Transkriptionsabbruch des CYPgst Gens führen beziehungsweise ein gestörtes Splicing verursachen könnten, und auf die mittels üblicher Verfahren zur Detektion von DNA-Punktmutationen (SNP-Analyse) getestet werden kann sind in der nachfolgenden Tabelle 1 zusammengestellt.

**Tabelle 1: Potentielle Punktmutationen im CYPgst Gen aus Beta vulgaris subsp. vulgaris, die zu einem vorzeitigen Transkriptionsabbruch des CYPgst Gens führen beziehungsweise ein gestörtes Splicing verursachen können.**

| **Position gemäß SEQ ID Nr. 1** | **Nukleotid** | **Mutation** | **Auswirkung der Mutation** |
|---|---|---|---|
| 1771 | G | T | STOP Codon |
| 1778 | T | A or G | STOP Codon |
| 1788 | T | A or G | STOP Codon |
| 1790 | T | A or G | STOP Codon |
| 1797 | T | A | STOP Codon |
| 1813 | A | T | STOP Codon |
| 1820 | T | A or G | STOP Codon |
| 1824 | C | A or G | STOP Codon |
| 1825 | C | T | STOP Codon |
| 1829 | G | A | STOP Codon |
| 1830 | G | A | STOP Codon |
| 1834 | A | T | STOP Codon |
| 1842 | C | A or G | STOP Codon |
| 1844 | T | A or G | STOP Codon |
| 1848 | C | A or G | STOP Codon |
| 1857 | C | A or G | STOP Codon |
| 1858 | A | T | STOP Codon |
| 1883 | G | A | STOP Codon |
| 1884 | G | A | STOP Codon |
| 1889 | T | A or G | STOP Codon |
| 1894 | G | T | STOP Codon |
| 1906 | C | T | STOP Codon |
| 1940 | C | A or G | STOP Codon |
| 1947 | T | A | STOP Codon |
| 1948 | G | T | STOP Codon |
| 1951 | A | T | STOP Codon |
| 1956 | T | A or G | STOP Codon |
| 1964 | T | A or G | STOP Codon |
| 1971 | C | A or G | STOP Codon |
| 2014 | G | T | STOP Codon |
| 2026 | G | T | STOP Codon |
| 2033 | T | A or G | STOP Codon |
| 2038 | C | T | STOP Codon |
| 2041 | C | T | STOP Codon |
| 2075 | T | A or G | STOP Codon |
| 2090 | T | A | STOP Codon |
| 2097 | C | A or G | STOP Codon |
| 2117 | T | A | STOP Codon |
| 2128 | G | T | STOP Codon |
| 2138 | G | A | STOP Codon |
| 2139 | G | A | STOP Codon |
| 2140 | A | T | STOP Codon |
| 2143 | A | T | STOP Codon |
| 2149 | A | T | STOP Codon |
| 2160 | C | A | STOP Codon |
| 2164 | G | T | STOP Codon |
| 2171 | T | A | STOP Codon |
| 2182 | A | T | STOP Codon |
| 2185 | C | T | STOP Codon |
| 2191 | G | T | STOP Codon |
| 2218 | G | T | STOP Codon |
| 2224 | C | T | STOP Codon |
| 2231 | T | A | STOP Codon |
| 2236 | C | T | STOP Codon |
| 2246 | T | A or G | STOP Codon |
| 2260 | A | T | STOP Codon |
| 2266 | A | T | STOP Codon |
| 2279 | T | A | STOP Codon |
| 2284 | G | T | STOP Codon |
| 2291 | T | A or G | STOP Codon |
| 2320 | A | T | STOP Codon |
| 2327 | T | A | STOP Codon |
| 2335 | A | T | STOP Codon |
| 2338 | C | T | STOP Codon |
| 2343 | C | A or G | STOP Codon |
| 2368 | C | T | STOP Codon |
| 2380 | G | T | STOP Codon |
| 2401 | G | T | STOP Codon |
| 2405 | T | A | STOP Codon |
| 2411 | G | A | STOP Codon |
| 2412 | G | A | STOP Codon |
| 2414 | T | A or G | STOP Codon |
| 2423 | T | A | STOP Codon |
| 2430 | C | A or G | STOP Codon |
| 2432 | T | A | STOP Codon |
| 2442 | T | A or G | STOP Codon |
| 2444 | T | A | STOP Codon |
| 2453 | G | A | STOP Codon |
| 2454 | G | A | STOP Codon |
| 2459 | G | A | STOP Codon |
| 2460 | G | A | STOP Codon |
| 2472 | T | A or G | STOP Codon |
| 2473 | G | T | STOP Codon |
| 2478 | T | A | STOP Codon |
| 2479 | G | T | STOP Codon |
| 2482 | A | T | STOP Codon |
| 2485 | A | T | STOP Codon |
| 2494 | G | T | STOP Codon |
| 2500 | G | T | STOP Codon |
| 2503 | A | T | STOP Codon |
| 2527 | A | T | STOP Codon |
| 2536 | G | T | STOP Codon |
| 2539 | G | T | STOP Codon |
| 2548 | A | T | STOP Codon |
| 2551 | G | T | STOP Codon |
| 2554 | A | T | STOP Codon |
| 2557 | A | T | STOP Codon |
| 2563 | A | T | STOP Codon |
| 2566 | G | T | STOP Codon |
| 2569 | G | T | STOP Codon |
| 2575 | G | T | STOP Codon |
| 2584 | G | T | STOP Codon |
| 2590 | G | T | STOP Codon |
| 2612 | T | A | STOP Codon |
| 2615 | T | A | STOP Codon |
| 2621 | T | A | STOP Codon |
| 2629 | G | T | STOP Codon |
| 2635 | G | T | STOP Codon |
| 2641 | G | T | STOP Codon |
| 2665 | A | T | STOP Codon |
| 2677 | C | T | STOP Codon |
| 2679 | G | A | Spleiß-Mutation |
| 2680 | G | A | Spleiß-Mutation |
| 2681 | T | A | Spleiß-Mutation |
| 3505 | A | C or G or T | Spleiß-Mutation |
| 3506 | G | A or C or T | Spleiß-Mutation |
| 3535 | C | A or G | STOP Codon |
| 3549 | G | T | STOP Codon |
| 3553 | G | A | STOP Codon |
| 3554 | G | A | STOP Codon |
| 3564 | G | T | STOP Codon |
| 3573 | A | T | STOP Codon |
| 3594 | A | T | STOP Codon |
| 3600 | C | T | STOP Codon |
| 3603 | C | T | STOP Codon |
| 3606 | G | T | STOP Codon |
| 3624 | G | T | STOP Codon |
| 3633 | C | T | STOP Codon |
| 3645 | G | T | STOP Codon |
| 3649 | C | A or G | STOP Codon |
| 3671 | C | A or G | STOP Codon |
| 3680 | T | A | STOP Codon |
| 3690 | G | T | STOP Codon |
| 3699 | C | T | STOP Codon |
| 3724 | T | A or G | STOP Codon |
| 3735 | G | T | STOP Codon |
| 3739 | C | A or G | STOP Codon |
| 3767 | T | A or G | STOP Codon |
| 3811 | T | A or G | STOP Codon |
| 3813 | G | T | STOP Codon |
| 3825 | A | T | STOP Codon |
| 3832 | G | A | STOP Codon |
| 3833 | G | A | STOP Codon |
| 3843 | G | T | STOP Codon |
| 3854 | C | A or G | STOP Codon |
| 3858 | G | T | STOP Codon |
| 3861 | A | T | STOP Codon |
| 3868 | G | A | STOP Codon |
| 3869 | G | A | STOP Codon |
| 3874 | T | A | STOP Codon |
| 3879 | G | T | STOP Codon |
| 3885 | A | T | STOP Codon |
| 3891 | G | T | STOP Codon |
| 3903 | G | T | STOP Codon |
| 3915 | A | T | STOP Codon |
| 3922 | T | A or G | STOP Codon |
| 3939 | A | T | STOP Codon |
| 3942 | A | T | STOP Codon |
| 3945 | A | T | STOP Codon |
| 3950 | T | A | STOP Codon |
| 3982 | T | A | STOP Codon |
| 3987 | G | T | STOP Codon |
| 3991 | T | A | STOP Codon |
| 4018 | G | A | STOP Codon |
| 4019 | G | A | STOP Codon |
| 4021 | T | A or G | STOP Codon |
| 4032 | G | T | STOP Codon |
| 4038 | A | T | STOP Codon |
| 4044 | G | T | STOP Codon |
| 4047 | G | T | STOP Codon |
| 4059 | A | T | STOP Codon |
| 4062 | G | T | STOP Codon |
| 4070 | T | A or G | STOP Codon |
| 4086 | A | T | STOP Codon |
| 4092 | C | T | STOP Codon |
| 4099 | T | A or G | STOP Codon |
| 4108 | T | A | STOP Codon |
| 4113 | A | T | STOP Codon |
| 4132 | T | A or G | STOP Codon |
| 4136 | T | A or G | STOP Codon |

### 3. Verwendung des CYPgst Gens bzw. -Lokus in der Hybridzüchtung

Wie bereits vorstehend beschrieben, wird der männlich sterile Phänotyp, den der gst-Locus hervorruft, in Resistenzzuchtprogrammen zum einfachen Durchkreuzen im Rahmen der rekurrenten Selektion eingesetzt. Vor der Klonierung des Gens im Rahmen der vorliegenden Erfindung und der damit verbundenen Entwicklung von genomischen Markern mussten wegen der erwarteten phänotypischen 3:1 Spaltung viermal mehr Pflanzen angezogen und ausgebracht werden, als benötigt. Diese Pflanzen mussten bei einsetzender Blüte innerhalb kurzer Zeit auf Sterilität bonitiert werden und fertile Individuen mussten entfernt werden, um Selbstbestäubungen zu vermeiden. Legt man mehrere tausend Pflanzen pro Jahr zu Grunde, ist diese manuelle Selektion sehr arbeitsaufwändig und auch fehleranfällig. Nunmehr werden erfindungsgemäß genomische Marker bereitgestellt, siehe Beispiel 2 und Abbildung 2 mit denen es beispielsweise möglich war, 30.000 Pflanzen zu testen und 7.500 männlich sterile Individuen zu selektieren, die anschließend gepflanzt wurden.

Es gibt ein beständiges Bemühen, Zuchtprogramme und Saatgutproduktion für Zuckerrüben zu vereinfachen und damit Kosten zu sparen. So werden kommerzielle Zuckerrüben gegenwärtig als Dreifach-Hybride erzeugt, um Saatgut von ausreichend hoher Qualität zu produzieren. Die Produktion von Hybriden in den Zuchtprogrammen, also im nicht-kommerziellen Bereich, ist ebenfalls kosten- und arbeitsintensiv und wird gegenwärtig durch Aufstellen von Trennwänden gewährleistet. Mit Hilfe des erfindungsgemäßen gst-Phänotyps und zugehörigen DNA Markern ist es nunmehr möglich - nach Einbringen des gst-Locus bzw. Mutation/Inhibierung des CYPgst Gens in die Zuchtprogramme -, männlich sterile Pflanzen vor dem Auspflanzen durch DNA-Marker zu selektieren und die Produktionsabläufe damit zu vereinfachen. Die parallele Entwicklung von multigermen Tester-Genotypen (MUS-Tester) würde sich damit ebenfalls erübrigen. Langfristig ist es ebenfalls denkbar, gegenwärtig verwendete CMS Technologie durch ein alternatives System mit dem die Samenelter-Komponente in einen männlich sterilen Zustand versetzt werden kann, beispielweise mittels dem vorstehend erwähnten und in Abbildung 5 gezeigten SPT-System, zu ersetzen. Dementsprechend ist es auch angemessen anzunehmen, das erfindungsgemäße CYPgst System in anderen Kultur-, insbesondere Nutzpflanzen Anwendung findet wie in der kommerziellen Produktion von Zweifachhybriden, wie im Mais. Im Mais (*Zea mays*) spielen *ms-Gene* eine große Rolle für die Entwicklung alternativer Systeme zur Herstellung von Hybridsaatgut. Sequenzanalysen ergaben, dass ein putatives Mais-Homolog zu *Bv*CYP*gst* existiert (GRMZM5g830329). Ebenso existiert eine große Anzahl von *ms*-Mutanten in Mais, von denen bislang nur ein Teil kloniert wurde. Mittels der vorliegenden Erfindung können nun die ms-Mutanten isoliert werden, der *ms*-Phänotyp auf eine Mutation bzw. Inhibierung des Mais-Homolog zu *Bv*CYP*gst* zurückzuführen ist und gezielt in der Saatgutproduktion eingesetzt werden. Ebenso findet die vorliegende Erfindung Einsatz in der Entwicklung einer Hybridkartoffel, beispielsweise um gezielt Mutationen in dem Kartoffelhomolog des *Bv*CYP*gst* Gens einzuführen und die so erworbene männliche Sterilität in der Kartoffel zu nutzen, um im Sinne des SPT-System eine diploide Hybridkartoffel zu entwickeln.

Schließlich ermöglicht die spezifische Expression des Gens *BvCYPgst* in Blüten und dem Tapetum eine biotechnologische Nutzung des Promotors, beispielsweise zur Expression einer Sense-/Antisense-RNA oder eines Ribozyms zur Inhibierung des *BvCYPgst* Gens oder zur Expression eines funktionellen CYP*gst* Proteins bzw. eines putativen Homolog, Analog oder Ortholog des *BvCYPgst* Gens zur Komplementation der Mutation und Restauration des männlich fertilen Phänotyps. Es wird davon ausgegangen, dass die Bereitstellung des Genlocus und der Nuklein- und Aminosäuresequenzen des *BvCYPgst* Gens nebst der genetischen Marker und die sich davon ableitenden Ausführungsformen eine wesentliche Vereinfachung und Kostenersparnis seitens der Zuchtprogramme mit sich bringt, da u.a. bereits eine frühzeitige Selektion steriler Individuen ermöglicht wird. Ebenso lässt sich damit eine logistische Vereinfachung und züchterische Erweiterung der beteiligten Programme erreichen.

## Patentansprüche

1. Pflanze der Art *Beta vulgaris,* die einen rezessiven, kernkodierten männlich sterilen Phänotyp zeigt, **dadurch gekennzeichnet, dass** der Phänotyp mit einer Mutation im endogenen Cytochrom P450 Oxidase Gen korreliert, **dadurch gekennzeichnet, dass** es sich bei dem nicht-mutierten Cytochrom P450 Oxidase Gen um ein Gen handelt ausgewählt aus der Gruppe bestehend aus:
(a) einer Nukleotidsequenz, die die in SEQ ID Nr.: 1 oder SEQ ID Nr.: 2 gezeigte Nukleotidsequenz oder ein funktionelles Fragment davon aufweist;
(b) einer Nukleotidsequenz, die die in SEQ ID Nr.: 3 gezeigte Aminosäuresequenz kodiert;
(c) einer Nukleotidsequenz, die in der Lage ist, an eine Nukleotidsequenz komplementär zu einer Nukleotidsequenz gemäß (a) oder (b) unter stringenten Bedingungen zu hybridisieren; und
(d) einer Nukleotidsequenz, die eine Aminosäuresequenz kodiert, die eine Sequenzidentität von mindestens 80% über die gesamte Aminosäuresequenz gemäß SEQ ID Nr.: 3 aufweist,
wobei es sich bei der Mutation um eine Deletion, Addition, Insertion oder Substitution in der kodierenden Nukleotidsequenz des Cytochrom P450 Oxidase Gens, in einem Splicing-Signal oder in einer regulatorischen Sequenz, vorzugsweise der Promotorsequenz, des Cytochrom P450 Oxidase Gens handelt, und
wobei die besagte Mutation die Transkription und/oder Translation eines funktionellen Cytochrom-P450-Oxidase-Proteins reduziert oder verhindert.

2. Pflanze nach Anspruch 1, die homozygot für die Mutation und männlich steril ist, wobei in der sterilen Pflanze die Ausbildung von funktionellem Pollen unterbunden ist.

3. Pflanze nach Anspruch 1, wobei es sich bei der Mutation um eine Deletion zwischen den Nukleotidpositionen 1560 und 2095 der SEQ ID Nr.: 1 handelt.

4. Pflanze nach Anspruch 1, wobei die Deletion durch Abwesenheit einer oder beider der Markerloci sle5983d14 (Amplifikationsprodukt der Primer mit SEQ ID Nr.: 4 und 5) und sle5983d17 (Amplifikationsprodukt der Primer mit SEQ ID Nr.: 6 und 7) und durch Anwesenheit eines ubiquitären Markers nachgewiesen werden kann.

5. Pflanze nach einem der Ansprüche 1-2, wobei das Gen in einem Segment auf Chromosom 1 zwischen den Markerloci sxn2151s01 und sle3305s02 lokalisiert ist, wobei die sxn2151s01-Markersequenz dargestellt in SEQ ID Nr.: 24 und die sle3305s02-Markersequenz dargestellt in SEQ ID Nr.: 26 das Vorhandensein des Locus, der die kernkodierte männliche Sterilität verursacht, anzeigen und die sxn2151s01-Markersequenz dargestellt in SEQ ID Nr.: 25 und die sle3305s02-Markersequenz dargestellt in SEQ ID Nr.: 27 die Referenzsequenz anzeigen.

6. Rekombinantes DNA-Molekül umfassend eine Nukleotidsequenz, die eine shRNA *(small hairpin RNA),* siRNA *(small interfering RNA),* Antisense-RNA, Sense-RNA oder doppelsträngige RNA kodiert, die nach Expression in einer Pflanzenzelle oder nach Einbringen in eine Pflanzenzelle zur Reduktion oder Inhibierung der Expression des funktionellen (nicht-mutierten) Cytochrom P450 Oxidase Gens wie in Anspruch 1 definiert, führt.

7. Pflanzenzelle enthaltend das rekombinante DNA-Molekül nach Anspruch 6.

8. Verfahren zur Herstellung einer männlich sterilen Pflanze der Art *Beta vulgaris,* **dadurch gekennzeichnet, dass** die Expression des Cytochrom P450 Oxidase Gens gemäß einer der Nukleotidsequenzen nach Anspruch 1 (a), (b), (c), oder (d) reduziert oder inhibiert wird, wobei das Verfahren einen Schritt des Einbringens des rekombinanten DNA-Moleküls nach Anspruch 6 umfasst, wobei besagter Schritt des Verfahrens eine Agrobacterium-Transformation, ein T-DNA-Tagging, oder eine Mutagenese mittels TILLING umfasst, oder einen Schritt der gezielten Mutagenese der Nukleotidsequenzen nach Anspruch 1 (a), (b), (c), oder (d) umfasst, infolgedessen die Expression des Gens beispielsweise durch RNAi oder Co-Suppression oder aufgrund der eingebrachten Mutation reduziert oder inhibiert wird.

9. Pflanze enthaltend Pflanzenzellen nach Anspruch 7 und/oder erhältlich durch ein Verfahren nach Anspruch 8.

10. Verfahren zur Restauration der Fertilität einer Pflanze nach einem der Ansprüche 1 bis 5 oder einer Pflanze nach Anspruch 9, umfassend das Einbringen eines funktionellen (nicht-mutierten) Cytochrom P450 Oxidase Gens in die Pflanze durch Transformation mittels einer rekombinanten DNA, die eine nicht-mutierte Nukleotidsequenz mit einer kodierenden Sequenz gemäß einem der Ansprüche 1 (a) - (d) umfasst, die operativ mit einem heterologen Promoter verknüpft ist, der vorzugsweise in der Lage ist, die Expression der Nukleotidsequenz spezifisch in geschlossenen Blüten und/oder Früchten zu steuern.

11. Organ, Pflanzenteil, Gewebe oder Zelle der Pflanze nach einem der Ansprüche 1 bis 5 oder 9 oder Samen oder Nachkommen der Pflanze nach einem der Ansprüche 1 bis 5 oder 9, wobei der Samen oder die Nachkommen die in einem der Ansprüche 1 bis 5 definierte Mutation und/oder das rekombinante DNA-Molekül nach Anspruch 6 aufweist.

12. Verfahren zur Identifizierung einer Pflanze nach einem der Ansprüche 1 bis 5 durch Nachweis der Mutation in dem Cytochrom P450 Oxidase Gen bzw. eines Markers, der mit der Mutation gekoppelt ist.

13. Verwendung eines Nukleinsäuremoleküls von mindestens 15 Nukleotiden Länge, das spezifisch an eine wie in Anspruch 1 definierte Nukleotidsequenz hybridisiert, und/oder eines Oligonukleotids, vorzugsweise mit einer Länge von maximal 50 Nukleotiden, das eine der folgenden Nukleotidsequenzen aufweist:
(i) SEQ ID Nr.: 4, 6 oder einem Komplement davon, oder
(ii) SEQ ID Nr.: 5, 7 oder einem Komplement davon, zur Identifizierung einer Pflanze nach einem der Ansprüche 1 bis 5.

## Claims

1. A plant of the species *Beta vulgaris,* showing a recessive, nuclear-encoded male sterile phenotype, **characterized in that** the phenotype is associated with a mutation in the endogenous cytochrome P450 oxidase gene, **characterized in that** the non-mutated cytochrome P450 oxidase gene is a gene selected from the group consisting of:
(a) a nucleotide sequence that has the nucleotide sequence shown in SEQ ID No.: 1 or SEQ ID No.: 2 or a functional fragment thereof;
(b) a nucleotide sequence encoding the amino acid sequence shown in SEQ ID No.: 3;
(c) a nucleotide sequence capable of hybridizing to a nucleotide sequence complementary to a nucleotide sequence according to (a) or (b) under stringent conditions; and
(d) a nucleotide sequence encoding an amino acid sequence that has a sequence identity of at least 80% over the entire amino acid sequence according to SEQ ID No.: 3,
wherein the mutation is a deletion, addition, insertion or substitution in the coding nucleotide sequence of the cytochrome P450 oxidase gene, in a splicing signal or in a regulatory sequence, preferably the promoter sequence of the cytochrome P450 oxidase gene, and
wherein said mutation in question reduces or prevents the transcription and/or translation of a functional cytochrome P450 oxidase protein.

2. The plant according to claim 1 which is homozygous for the mutation and male-sterile, wherein the formation of functional pollen is inhibited in the sterile plant.

3. The plant according to claim 1, wherein the mutation is a deletion between the nucleotide positions 1560 and 2095 of SEQ ID No.: 1.

4. The plant according to claim 1, wherein the deletion can be detected by the absence of one or both of the marker loci sle5983d14 (amplification product of the primers with SEQ ID Nos.: 4 and 5) and s1e5983d17 (amplification product of the primers with SEQ ID Nos.: 6 and 7) and by the presence of a ubiquitous marker.

5. The plant according to any one of claims 1-2, wherein the gene is localized in a segment on chromosome 1 between the marker loci sxn2151s01 and sle3305s02, wherein the sxn2151s01 marker sequence shown in SEQ ID No.: 24 and the sle3305s02 marker sequence shown in SEQ ID No.: 26 indicate the presence of the locus causing the nuclear-encoded male sterility, and the sxn2151s01 marker sequence shown in SEQ ID No.: 25 and the SLE3305S02 marker sequence shown in SEQ ID No.: 27 show the reference sequence.

6. Recombinant DNA molecule comprising a nucleotide sequence that encodes an shRNA *(small hairpin RNA),* siRNA *(small interfering RNA),* antisense RNA, sense RNA or double-stranded RNA which, after expression in a plant cell or after introduction into a plant cell, results in the reduction or inhibition of the expression of the functional (non-mutated) cytochrome P450 oxidase gene as defined in claim 1.

7. Plant cell comprising the recombinant DNA molecule according to claim 6.

8. Method of producing a male sterile plant of the species *Beta vulgaris,* **characterized in that** the expression of the cytochrome P450 oxidase gene is reduced or inhibited according to one of the nucleotide sequences according to claim 1 (a), (b), (c), or (d), wherein the method comprises a step of introduction of the recombinant DNA molecule according to claim 6, wherein said step of the method comprises an agrobacterium transformation, a T-DNA tagging, or mutagenesis by TILLING, or a step of targeted mutagenesis of the nucleotide sequences according to claim 1 (a), (b), (c), or (d), as a result of which the expression of the gene is reduced or inhibited, e.g., by RNAi or co-suppression or due to the introduced mutation.

9. Plant comprising plant cells according to claim 7 and/or obtained by a method according to claim 8.

10. A method for restoring the fertility of a plant according to any one of claims 1 to 5 or a plant according to claim 9, comprising the introduction of a functional (non-mutated) cytochrome P450 oxidase gene into the plant by transformation by means of a recombinant DNA comprising a non-mutated nucleotide sequence having a coding sequence according to any one of claims 1 (a)-(d) which is surgically linked to a heterologous promoter, which is preferably able to control the expression of the nucleotide sequence specifically in closed flowers and/or fruits.

11. An organ, plant part, tissue or cell of the plant according to any one of claims 1 to 5 or 9 or seed or descendants of the plant according to any one of claims 1 to 5 or 9, wherein the seed or descendants having the mutation defined in any one of claims 1 to 5 and/or the recombinant DNA molecule according to claim 6.

12. A method for identifying a plant according to any one of claims 1 to 5 by demonstrating the mutation in the cytochrome P450 oxidase gene or a marker coupled with the mutation.

13. Use of a nucleic acid molecule of at least 15 nucleotides in length which hybridises specifically to a nucleotide sequence as defined in claim 1 and/or an oligonucleotide, preferably having a length of no more than 50 nucleotides, having one of the following nucleotide sequences:
(i) SEQ ID No.: 4, 6 or a complement thereof, or
(ii) SEQ ID No.: 5, 7 or a complement thereof, for identifying a plant according to any of claims 1 to 5.

## Revendications

1. Plante de l'espèce *Beta vulgaris,* qui présente un phénotype mâle stérile récessif, codé par le noyau, **caractérisée en ce que** le phénotype est corrélé avec une mutation dans le gène endogène cytochrome P450 oxydase, **caractérisée en ce que** le gène cytochrome P450 oxydase non muté est un gène choisi dans le groupe constitué de :
(a) une séquence nucléotidique qui comprend la séquence nucléotidique représentée dans SEQ ID n° : 1 ou SEQ ID n° : 2 ou un fragment fonctionnel de celle-ci ;
(b) une séquence nucléotidique qui code pour la séquence d'acides aminés représentée dans SEQ ID n° : 3 ;
(c) une séquence nucléotidique qui est capable de s'hybrider à une séquence nucléotidique complémentaire à une séquence nucléotidique selon (a) ou (b) dans des conditions stringentes ; et
(d) une séquence nucléotidique qui code pour une séquence d'acides aminés qui présente une identité de séquence d'au moins 80 % sur toute la séquence d'acides aminés selon SEQ ID n° : 3,
dans laquelle la mutation est une délétion, addition, insertion ou substitution dans la séquence nucléotidique codante du gène cytochrome P450 oxydase, dans un signal d'épissage ou dans une séquence régulatrice, de préférence la séquence promotrice, du gène cytochrome P450 oxydase, et
dans laquelle ladite mutation réduit ou empêche la transcription et/ou la traduction d'une protéine cytochrome P450 oxydase fonctionnelle.

2. Plante selon la revendication 1, qui est homozygote pour la mutation et mâle stérile, dans laquelle, dans la plante stérile, la formation de pollen fonctionnel est empêchée.

3. Plante selon la revendication 1, dans laquelle la mutation est une délétion entre les positions nucléotidiques 1560 et 2095 de SEQ ID n° : 1.

4. Plante selon la revendication 1, dans laquelle la délétion peut être détectée par l'absence d'un ou des deux loci marqueurs sle5983d14 (produit d'amplification des amorces avec SEQ ID n° : 4 et 5) et sle5983d17 (produit d'amplification des amorces avec SEQ ID n° : 6 et 7) et par la présence d'un marqueur ubiquitaire.

5. Plante selon l'une des revendications 1 et 2, dans laquelle le gène est localisé dans un segment sur le chromosome 1 entre les loci marqueurs sxn2151s01 et sle3305s02, où la séquence marqueur sxn2151s01 représentée dans SEQ ID n° : 24 et la séquence marqueur sle3305s02 représentée dans SEQ ID n° : 26 indiquent la présence du locus qui cause la stérilité mâle codée par le noyau et la séquence marqueur sxn2151s01 représentée dans SEQ ID n° : 25 et la séquence marqueur sle3305s02 représentée dans SEQ ID n° : 27 indiquent la séquence de référence.

6. Molécule d'ADN recombinant comprenant une séquence nucléotidique qui code pour un shARN *(petit ARN en épingle à cheveux*), siARN (*petit ARN interférent*), ARN antisens, ARN sens ou ARN double brin qui, après expression dans une cellule végétale ou après introduction dans une cellule végétale, conduit à la réduction ou à l'inhibition de l'expression du gène cytochrome P450 oxydase fonctionnel (non muté) tel que défini dans la revendication 1.

7. Cellule végétale contenant la molécule d'ADN recombinant selon la revendication 6.

8. Procédé de production d'une plante mâle stérile de l'espèce *Beta vulgaris,* **caractérisé en ce que** l'expression du gène cytochrome P450 oxydase selon l'une des séquences nucléotidiques selon la revendication 1 (a), (b), (c), ou (d) est réduite ou inhibée, dans lequel le procédé comprend une étape d'introduction de la molécule d'ADN recombinant selon la revendication 6, dans lequel ladite étape du procédé comprend une transformation par Agrobacterium, un marquage par T-ADN, ou une mutagénèse par Tilling (ciblage de lésions locales dans les génomes), ou comprend une étape de mutagénèse ciblée des séquences nucléotidiques selon la revendication 1 (a), (b), (c), ou (d), par conséquent l'expression du gène est réduite ou inhibée par exemple par ARNi ou co-suppression ou en raison de la mutation introduite.

9. Plante contenant des cellules végétales selon la revendication 7 et/ou pouvant être obtenue par un procédé selon la revendication 8.

10. Procédé de restauration de la fertilité d'une plante selon l'une des revendications 1 à 5 ou d'une plante selon la revendication 9, comprenant l'introduction d'un gène cytochrome P450 oxydase fonctionnel (non muté) dans la plante par transformation au moyen d'un ADN recombinant qui comprend une séquence nucléotidique non mutée avec une séquence codante selon l'une des revendications 1 (a) à (d), qui est liée de manière opérationnelle à un promoteur hétérologue qui est de préférence capable de commander l'expression de la séquence nucléotidique spécifiquement dans les fleurs fermées et/ou les fruits.

11. Organe, partie de plante, tissu ou cellule de la plante selon l'une des revendications 1 à 5 ou 9 ou graines ou descendants de la plante selon l'une des revendications 1 à 5 ou 9, dans lesquels la graine ou les descendants présentent la mutation définie dans l'une des revendications 1 à 5 et/ou la molécule d'ADN recombinant selon la revendication 6.

12. Procédé d'identification d'une plante selon l'une des revendications 1 à 5 par détection de la mutation dans le gène cytochrome P450 oxydase ou d'un marqueur qui est couplé à la mutation.

13. Utilisation d'une molécule d'acide nucléique d'au moins 15 nucléotides de longueur, qui s'hybride de manière spécifique à une séquence nucléotidique telle que définie dans la revendication 1, et/ou d'un oligonucléotide, de préférence d'une longueur maximale de 50 nucléotides, qui présente l'une des séquences nucléotidiques suivantes :
(i) SEQ ID n° : 4, 6 ou un complément de celle-ci, ou
(ii) SEQ ID n° : 5, 7 ou un complément de celle-ci, pour l'identification d'une plante selon l'une des revendications 1 à 5.
